# EUROPÄISCHE PATENTSCHRIFT

(11) **EP 3 645 674 B1**
(45) Veröffentlichungstag und Bekanntmachung des Hinweises auf die Patenterteilung: **04.05.2022**
(21) Anmeldenummer: 19706482.7
(22) Anmeldetag: 15.02.2019
(51) Int. Cl.: C10L 1/06, C07C 1/20, C10G 3/00

(54) **VERFAHREN UND ANLAGE ZUR HERSTELLUNG EINES SYNTHETISCHEN BENZINS**
METHOD AND INSTALLATION FOR PRODUCING A SYNTHETIC GASOLINE
PROCÉDÉ ET INSTALLATION DE PRODUCTION D'UNE ESSENCE SYNTHÉTIQUE

(30) Priorität: 16.02.2018 DE 102018103552
(43) Veröffentlichungstag der Anmeldung: 06.05.2020
(73) Patentinhaber: Chemieanlagenbau Chemnitz GmbH, 09111 Chemnitz (DE)
(72) Erfinder: ENGELMANN, Joachim, 09221 Neukirchen-Adorf (DE); ENGELMANN, Jörg, 09117 Chemnitz (DE); SEIDEL, Petra, 09669 Frankenberg (DE)
(74) Vertreter: Kailuweit & Uhlemann Patentanwälte Partnerschaft mbB
(86) Internationale Anmeldenummer: PCT/EP2019/053801
(87) Internationale Veröffentlichungsnummer: WO 2019/158687

(56) Entgegenhaltungen:
- WO-A1-2018/007484
- DE-A1-102009 046 790
- US-A- 4 035 430
- US-A1- 2016 102 032
- US-A1- 2016 122 256

## Beschreibung

Die Erfindung betrifft ein Verfahren und eine Anlage zur Herstellung eines synthetischen Benzins mit hohem iso-Paraffingehalt und mit niedrigem Aromatengehalt, insbesondere eines hochoktanigen Benzins zur Verwendung als Ottokraftstoff, aus Alkoholen.

Verfahren zur Herstellung von Benzin aus Alkoholen sind bekannt. So wurde in den 70-iger Jahren von der Mobil Oil Corporation der MTG-Prozess ("methanol to gasoline") der Synthese von hochoktanigem Benzin aus Methanol entwickelt, der unter anderem in US 3,894,102 A beschrieben ist. Dabei wird in der ersten Stufe aus einem Gemisch aus Kohlenmonoxid und Wasserstoff an einem Katalysatorgemisch, bestehend aus einem Methanolsynthesekatalysator und einem sauren Dehydratisierkatalysator, ein Produkt synthetisiert, welches hauptsächlich Dimethylether (DME) enthält. Nach Produktkühlung und -separation wird in der zweiten Stufe der so erzeugte Dimethylether mit einem kristallinen Aluminosilikat- Zeolithkatalysator kontaktiert und es entsteht ein Produkt, das hauptsächlich aus flüssigen aromatischen Kohlenwasserstoffen des Benzinsiedebereiches (C₅ bis 400 °F) besteht.

Die Umwandlung von Methanol in ein Kohlenwasserstoffprodukt kann in Festbettreaktoren (siehe z. B. US 3,998,899 A, US 3,931,349 A und US 4,035,430 A) und in Reaktoren mit Katalysatorfließbetten (siehe z. B. US 4,071,573 A und US 4,138,440 A) stattfinden. Beim MTG-Prozess wird das Methanol dehydratisiert, wobei meist ein γ-Aluminiumoxid-Katalysator verwendet wird und ein Gleichgewichtsgemisch aus Methanol, Dimethylether und Wasser entsteht. Dieses Gemisch wird dann in einem ZSM-5 (Zeolith) - Katalysatorbett in ein Kohlenwasserstoffprodukt eines Siedebereiches von leichten Kohlenwasserstoffen bis Benzin und Wasser umgewandelt.

Das Hauptproblem, das es bei dem MTG-Prozess zu lösen gilt, ist das Problem der Temperaturkontrolle im Reaktor. So werden aufgrund der stark exothermen Reaktion bei der Umwandlung von Methanol zu Kohlenwasserstoffen ca. 1740 kJ pro umgesetztes Kilogramm Methanol frei. In einem adiabaten Reaktor würde das zu einem Temperaturanstieg um ca. 650 °C führen, was eine extrem schnelle Katalysatoralterung bzw. sogar dessen Zerstörung bewirken würde. Außerdem würde der adiabate Temperaturanstieg zur Bildung von unerwünschten Nebenprodukten führen.

Eine Methode zur Vermeidung eines zu starken Temperaturanstiegs im Katalysatorbett ist in US 3,931,349 beschrieben. Dabei wird die starke Exothermie bei der Reaktion von Methanol zu Benzinkohlenwasserstoffen in einem System von parallel angeordneten Katalysatorbetten kontrolliert. Das Einsatzmethanol wird verdampft und in der ersten Stufe durch Kontakt mit einem γ-Aluminiumoxid-Katalysator in ein Gemisch aus Dimethylether, Methanol und Wasser umgewandelt, wobei 15 bis 20 % der Gesamtwärme der Reaktion von Methanol zu Benzinkohlenwasserstoffen freigesetzt werden.

Das Reaktionsprodukt der Dehydratisierung von Methanol wird mit den leichten Kohlenwasserstoffgasen < C₅ aus der zweiten Reaktionsstufe der Umwandlung verdünnt. Diese werden vom Reaktionsprodukt der Kohlenwasserstoffsynthese nach dessen Abkühlung abgetrennt, komprimiert und vorgeheizt und anschließend mit dem Einsatzstrom des Reaktors vermischt. Das Kreislaufgas kühlt das Katalysatorbett während der exothermen Reaktion der Oxygenate zu Kohlenwasserstoffen an einem kristallinen ZSM-5. Die Reaktion der Kohlenwasserstoffsynthese wird dabei bei möglichst geringem Druck durchgeführt, um die unerwünschte Bildung hochmolekularer aromatischer Verbindungen, wie Durol, zu minimieren. Die Raumgeschwindigkeit, bezogen auf reines Methanol, beträgt 1 h⁻¹ und der Partialdruck des Methanols 1 bar. Der niedrige Partialdruck des Reaktanden begrenzt die Bildung unerwünschter schwerer Aromaten.

In US 3,998,899 A wird ein Prozess der Umwandlung von C₁- bis C₃- Alkoholen, bevorzugt von Methanol, in Kohlenwasserstoffe des Benzinsiedebereiches beschrieben. In einer ersten katalytischen Stufe werden aus den Alkoholen Etherprodukte gebildet und in einer zweiten katalytischen Stufe an einem kristallinen ZSM-5 Zeolith Komponenten des Benzinsiedebereiches.

In US 4,814,535 A wird ein Verfahren beschrieben, das es ermöglicht, den Umsatzgrad in einer Reaktionsperiode, trotz der allmählichen Desaktivierung des Katalysators durch Koksablagerungen, konstant zu halten. Dazu wird die Eintrittstemperatur in den Reaktor der Kohlenwasserstoffsynthese schrittweise um 3 bis 9 °C angehoben, wobei die Oktanzahl konstant bleibt.

Bei den oben beschriebenen Methoden ist ein sehr hohes Recycle-Verhältnis (Kreislaufgas zu Einsatzstrom) erforderlich, wodurch der Prozess durch das notwendige Kühlen, Komprimieren und das anschließende Aufheizen des Kreislaufgases aufwendig wird.

Wird zur Erhöhung der Effektivität des Prozesses das Kreislaufgas- zu Einsatzstrom-Verhältnis verringert, bedingt das eine Erhöhung der Temperaturdifferenz über dem Katalysatorbett, was sich nicht nur in einer schnelleren Alterungsrate des Katalysators widerspiegelt, sondern auch in der Erhöhung des Wasserdampf-Partialdrucks (aufgrund des größeren Wasserdampfanteils bei Senkung des Kreislaufgasanteils). Ein hoher Wasserdampf-Partialdruck wirkt sich negativ auf die Katalysatorlebensdauer aus, insbesondere bei gleichzeitig hohen Reaktionstemperaturen.

In US 4,404,414 A wird deshalb eine Methode der Wärmeabfuhr beschrieben, durch die bei reduzierten Recycle- Verhältnissen die gewünschten Temperaturen im Katalysatorbett eingehalten werden können. Die Anordnung der Reaktoren wird dabei so gestaltet, dass die Reaktoren für den Oxygenat- Einsatz parallel angeordnet sind, für das Kreislaufgas jedoch in Reihe. Durch die spezielle Reaktoranordnung wird erreicht, dass die gesamte Recyclegasmenge bei gleichen Temperaturdifferenzen über den Katalysatorbetten auf die Hälfte reduziert werden kann.

In US 4,788,369 A wird die LPG-Fraktion (Propan, n-Butan und iso-Butan und geringe Mengen an leichten Olefinen) vom Reaktionsprodukt getrennt und zum Reaktor rückgeführt, um die Reaktionswärme abzuführen. Ein wesentlicher Vorteil der Verwendung der LPG-Fraktion als Recyclestrom gegenüber der sonst üblichen Rückführung der leichten Kohlenwasserstoffgase ist die Verbesserung der Wirtschaftlichkeit des Verfahrens. Es wurde ein geringer Anstieg der Benzinausbeute (um ca. 1 %) festgestellt, da sich die im rückgeführten LPG-Strom enthaltenen Olefine zu Benzinkohlenwasserstoffen umgesetzt haben, wobei sich die Normalparaffine nicht umgesetzt haben.

In US 4,035,430 A wird zur Begrenzung des Temperaturanstiegs über dem Katalysatorbett der Kohlenwasserstoffsynthese der Einsatzstoff (Methanol oder das Produkt der DME-Synthese) mit leichten Kohlenwasserstoffen < C₅ und teilweise auch mit Durol verdünnt, welches aus der C₁₀₊-Kohlenwasserstofffraktion durch Kristallisation abgetrennt wurde. Durch die Rückführung von Durol verringerte sich im Vergleich mit dem Prozess ohne Durolrückführung in einem niedrigeren T-Bereich (< 430 °C) der Aromatengehalt im Produkt (Aromaten außer Durol), während er sich in einem höheren Temperaturbereich (> 430 °C) erhöhte. Durch die Erhöhung des Aromatengehalts im Produkt bei höheren Temperaturen (> 430 °C) konnte auf diese Weise auch die Benzinselektivität gesteigert werden.

In WO 2011/061198 A1 wird ein zweistufiges Verfahren und eine entsprechende Anlage zur Erzeugung von Kohlenwasserstoffen beschrieben. Ausgehend von Synthesegas wird im ersten Schritt Methanol erzeugt, das dann im zweiten Schritt zu Benzinkohlenwasserstoffen und Wasser umgesetzt wird. Das Verfahren wird insbesondere durch eine Aufreinigung des erhaltenen Wassers gekennzeichnet. Darin enthaltenes nicht umgesetztes Methanol wird katalytisch zu Synthesegas umgesetzt. Des Weiteren ist eine Rückführung des so erhaltenen sowie des nicht umgesetzten Synthesegases vorgesehen.

In US 4,058,576 A wird eine Methode zur effizienteren Abführung der frei werdenden Wärme bei der Umsetzung von Methanol zu Benzin durch Aufteilung der Zwischenreaktionen auf 3 aufeinander folgende Prozessschritte beschrieben. Dabei werden die Reaktionsstufen der Methanolumwandlung zu Dimethylether (DME), der Umwandlung des DME's in Olefine und der Olefinumwandlung in Benzinkohlenwasserstoffe in gesonderten Katalysatorbetten durchgeführt.

Auch in US 4,052,479 A wird ein mehrstufiger Prozess zur Umsetzung von Alkoholen in Benzin und Olefinkohlenwasserstoffe beschrieben. Dazu wird zunächst in einem Zwischenschritt DME isoliert und dieses bei sehr kurzen Kontaktzeiten bevorzugt zu Olefinkohlenwasserstoffen und Benzin umgewandelt. Kurze Kontaktzeiten werden bei sehr hohen Belastungen mit einer Raumgeschwindigkeit in einem Bereich von 10 bis 2000 h⁻¹ ("Liquid hourly space velocity"-LHSV), bevorzugt von 50 bis 1000 h⁻¹, erreicht. Die hohe Belastung ist gleichzeitig ein Nachteil des Prozesses.

In US 4,387,263 A wird deshalb ein Prozess zur Erzeugung von Benzin und C₂- bis C₄-Olefinen aus einem Gemisch aus Methanol und optional Wasserdampf beschrieben, bei welchem hohe Olefinausbeuten schon bei wesentlich niedrigeren LHSV erreicht werden.

Auch DE 10 2005 048 931 A1 offenbart ein Verfahren zur Herstellung von C₂ - C₄ - Olefinen aus Methanol oder Dimethylether. Dabei wird ein Eduktgemisch aus Alkohol, Dimethylether und Wasser in einem Reaktor über ein Katalysatorbett geleitet und zu einem Produktgemisch umgesetzt, welches im ersten Schritt in ein C₅₋ - olefinreiches Gemisch, ein C₅₊ - kohlenwasserstoffreiches Gemisch und eine wässrige Phase aufgetrennt wird. Durch einen hohen Massenanteil an Wasser im Eduktgemisch von 20 bis 91%, gemessen an der Gesamtmasse des Alkohol-Wasser-Gemischs, gelingt es, einen hohen Anteil an niederen Olefinen zu generieren.

DE 10 2009 046 790 A1 offenbart ein Verfahren zur Herstellung von Benzin aus Synthesegas. Es wird auf hohe Benzinselektivität mit möglichst wenig umweltschädigenden Nebenprodukten, wie beispielsweise aromatenreichem Schwerbenzin, abgezielt. Allerdings ist die Beherrschung über die Exothermie nicht optimal und es treten auch hier ungewollte Temperaturspitzen auf, was zu einem veränderten Produktspektrum führt. Der Gehalt an iso-Paraffinen im Benzin ist damit zu niedrig.

Aufgabe der Erfindung ist die Entwicklung eines Verfahrens und einer entsprechenden Anlage zur Erzeugung von synthetischem Benzin aus Alkoholen, das einen hohen iso-Paraffingehalt und einen niedrigen Aromatengehalt aufweist. Die Anlage soll diese Synthese in möglichst energiesparender Weise erlauben.

Gelöst wird die Aufgabe durch ein Verfahren zur Herstellung eines synthetischen Benzins (in Form einer Stabilbenzinfraktion) mit hohem iso-Paraffingehalt im Bereich 50-65 Ma% und mit niedrigem Aromatengehalt im Bereich 20-35 Ma% mit den Schritten:
I. katalytische Umsetzung von Einsatzalkohol in einem Einsatzgas in ein Produktgemisch, enthaltend Wasser und ein Kohlenwasserstoffgemisch aus Olefinen, n-Paraffinen, iso-Paraffinen, Aromaten und Naphthenen, in einem isothermen Rohrreaktor, enthaltend einen Katalysator,
   wobei die Reaktionstemperatur im Rohrreaktor zwischen 300 und 370 °C liegt, wobei der Einsatzalkohol einen Wasseranteil von weniger als 20 Ma.-% aufweist.
II. Auftrennung des in Schritt **I)** erhaltenen Produktgemisches in
   - eine flüssige Kohlenwasserstoffphase,
   - eine wässrige Phase, enthaltend den nicht umgesetzten Alkohol, und
   - eine Gasphase, enthaltend C₁- bis C₅- Kohlenwasserstoffe,
III. Rückführung der in Schritt **II**) erhaltenen Gasphase zu Schritt I),
IV. Auftrennung der in Schritt **II**) erhaltenen flüssigen Kohlenwasserstoffphase in
   - eine olefinhaltige C₃- und C₄-Kohlenwasserstofffraktion und
   - eine Benzinkohlenwasserstofffraktion (= C₅₊-Fraktion), enthaltend eine durolhaltige schwere Aromatenfraktion,
V. Auftrennung der in Schritt **IV)** erhaltenen Benzinkohlenwasserstofffraktion in eine durolhaltige schwere Aromatenfraktion und eine Stabilbenzinfraktion,
   wobei in Schritt **I)** die Alkoholbelastung des Katalysators 2 bis 5 m³_{Alkohol/}hm³_{Kat} beträgt,
   wobei zur Erzeugung eines iso-Paraffin-reichen Benzins der Katalysator im Eintrittsbereich des Alkohols partiell mit 5-30 Massen-% Inertmaterial, bezogen auf die Gesamtmasse an Katalysator und Inertmaterial, verdünnt ist, und
   wobei die in Schritt **IV)** abgetrennte olefinhaltige C₃- bis C₄-Kohlenwasserstofffraktion als Einsatzstoff zu Schritt **I)** zurückgeführt wird,
   wobei in Schritt **I)** der Stoffmengenanteil des umzusetzenden Alkohols am Rohrreaktoreintritt 25 bis 50 Mol-% umfasst, gemessen an der Gesamtstoffmenge des Einsatzgases, umfassend den Einsatzalkohol und rückgeführte Komponenten.

Als Einsatzalkohol wird der in das Verfahren eingesetzte Roh-Alkohol bezeichnet. Dem Fachmann ist bekannt, dass Alkohol, insbesondere Alkohol von technischer Qualität, noch einen Anteil an Wasser aufweisen kann.

In einer Ausführungsform ist der Einsatzalkohol ein Alkohol-Wasser-Gemisch.

Erfindungsgemäß weist der Einsatzalkohol einen Wasseranteil von weniger als 20 Ma.-%, bevorzugt weniger als 15 Ma.-%, insbesondere weniger als 5 Ma.-% auf.

Ma.-% bezeichnet den Massenanteil an der Gesamtmasse in %.

In einer Ausführungsform ist der Massenanteil an Wasser im Einsatzalkohol mindestens 1 % gemessen an der Gesamtmasse des Einsatzalkohols.

In einer Ausführungsform beträgt der Massenanteil an Wasser im Einsatzalkohol 1 bis < 20 %, bevorzugt 1 bis < 15 % der Gesamtmasse des Einsatzalkohols.

In einer Ausführungsform ist der Einsatzalkohol wasserfrei.

Der Einsatzalkohol wird in flüssiger oder gasförmiger Form in das Kreislaufgas eingespeist.

In einer Ausführungsform ist das Kreislaufgas die in Schritt **III)** zum Reaktor in Schritt **I)** rückgeführte Gasphase.

Das Kreislaufgas wird zusammen mit dem Einsatzalkohol auf Reaktionstemperatur aufgeheizt. Die erhaltene Mischung wird gasförmig in den Reaktor eingespeist. Diese Mischung wird als Einsatzgas bezeichnet.

Erfindungsgemäß erfolgt die katalytische Umsetzung von Einsatzalkohol in einem Einsatzgas in ein Produktgemisch, enthaltend Wasser und ein Kohlenwasserstoffgemisch aus Olefinen, n-Paraffinen, iso-Paraffinen, Aromaten und Naphthenen, in einem isothermen Rohrreaktor, enthaltend einen Katalysator, wobei das Einsatzgas einen Einsatzalkohol mit einem Wasseranteil von weniger als 20 Ma.-%, gemessen an der Gesamtmasse des Einsatzalkohols, enthält.

In einer Ausführungsform enthält das Einsatzgas Inertgas und Einsatzalkohol.

In einer Ausführungsform enthält das Einsatzgas Einsatzalkohol und rückgeführte Komponenten.

Vorteilhaft erhält man durch das erfindungsgemäße Verfahren in Schritt **I)** eine Kohlenwasserstofffraktion mit einem geringen Anteil an Olefinen. In einer Ausführungsform beträgt der Massenanteil der Olefine im gebildeten Kohlenwasserstoffgemisch weniger als 20%, bevorzugt weniger als 10 %, besonders bevorzugt weniger als 5%, insbesondere weniger als 2% der Gesamtmasse des gebildeten Kohlenwasserstoffgemischs.

In einer Variante V1 des erfindungsgemäßen Verfahrens enthält das Verfahren die Schritte
a) katalytische Umsetzung von Alkohol in ein Produktgemisch, enthaltend Wasser und ein Kohlenwasserstoffgemisch aus Olefinen, n-Paraffinen, iso-Paraffinen, Aromaten und Naphthenen, in einem Reaktor, enthaltend einen Katalysator
b) Auftrennung des in Schritt a) erhaltenen Produktgemisches in
   - eine flüssige Kohlenwasserstoffphase,
   - eine wässrige Phase, enthaltend den nicht umgesetzten Alkohol und
   - eine Gasphase, enthaltend C₁- bis C₅- Kohlenwasserstoffe
c) Abtrennung des nicht umgesetzten Alkohols aus der in Schritt b) erhaltenen wässrigen Phase
d) Rückführung des in Schritt c) abgetrennten Alkohols als Einsatzstoff zu Schritt a)
e) Teilung der Gasphase aus Schritt b) in einen ersten und einen zweiten Teil und Rückführung des ersten Teils der in Schritt b) erhaltenen Gasphase zu Schritt a)
f) Abtrennung von C₃- bis C₅- Kohlenwasserstoffen aus dem zweiten Teil der Gasphase aus Schritt e)
g) Vereinen der in Schritt f) erhaltenen C₃- bis C₅- Kohlenwasserstoffe mit der in Schritt b) erhaltenen flüssigen Kohlenwasserstoffphase
h) Auftrennung der in Schritt g) erhaltenen flüssigen Kohlenwasserstoffphase in
   - eine C₃- bis C₄- Kohlenwasserstofffraktion und
   - eine Benzinkohlenwasserstofffraktion (= C₅₊-Fraktion), enthaltend eine durolhaltige schwere Aromatenfraktion
i) Rückführung der in Schritt h) abgetrennten C₃- bis C₄-Fraktion als Einsatzstoff zu Schritt a)
j) Auftrennung der in Schritt h) erhaltenen Benzinkohlenwasserstofffraktion in eine durolhaltige schwere Aromatenfraktion und eine Stabilbenzinfraktion.

Im Sinne der Erfindung umfasst der Begriff Alkohol organische Verbindungen R-OH, die mindestens eine OH-Gruppe (Hydroxygruppe) enthalten, die an einen Alkylrest (R) gebunden ist. Unter "Alkohol" versteht man im Sinne der Erfindung sowohl einen einzelnen Alkohol als auch eine Mischung mehrerer Alkohole. Im weiteren Verlauf wird zum besseren Verständnis der eingesetzte Alkohol oder die Mischung mehrerer Alkohole "Einsatzalkohol" genannt. Im Einsatzalkohol kann Wasser enthalten sein.

Die katalytische Umsetzung erfolgt in mehreren Reaktionsschritten. Aus dem Alkohol entsteht ein Ether, daraus im weiteren Verlauf Olefine. In weiteren Folgereaktionen bilden sich dann verschiedene Kohlenwasserstoffe, bestehend aus Olefinen, n-Paraffinen, iso-Paraffinen, Aromaten und Naphthenen. Bei nicht vollständigem Umsatz sind außer dem Einsatzalkohol auch Zwischenprodukte der Reaktion, wie Ether und niedere Olefine (Ethylen, Propylen, Butylen), im Produkt enthalten.

Ether im Sinne der Erfindung sind organische Verbindungen, die eine Ethergruppe R-O-R - ein Sauerstoffatom (O), das mit Alkylresten (R) substituiert ist, enthalten. Ether entstehen aus Alkoholen, bevorzugt an einem sauren Katalysator, durch deren Dehydratisierung (Wasserabspaltung). In Abhängigkeit vom eingesetzten Alkohol entstehen unterschiedliche Ether als Zwischenprodukte. Aus Methanol entsteht Dimethylether, aus Ethanol Diethylether und aus Propanol Dipropylether.

Der Alkohol ist zweckmäßigerweise und bevorzugt ausgewählt aus Verbindungen, enthaltend mindestens eine OH-Gruppe, die an einen Alkylrest mit 1 bis 3 C-Atomen gebunden ist. Der Alkylrest kann sowohl verzweigt als auch unverzweigt sein.

Bevorzugt ist der Alkohol ausgewählt aus Methanol, Ethanol, Isopropylalkohol (2-Propanol), n-Propanol (1-Propanol), besonders bevorzugt ist der Alkohol Methanol.

Der Einsatzalkohol kann zunächst entsprechend dem Stand der Technik synthetisiert werden. Vorteilhaft muss das im Reaktionsprodukt enthaltene Wasser (z. B. im Rohmethanol) im erfindungsgemäßen Verfahren vor der Einspeisung in den Reaktor nicht abgetrennt werden, sondern kann zusammen mit dem Alkohol in den Prozess eingespeist werden. Vorteilhaft kann damit eine Destillationsstufe eingespart werden.

Erfindungsgemäß enthält der Einsatzalkohol einen Wasseranteil von weniger als 20 Ma.-%, gemessen an der Gesamtmasse des Alkohol-Wasser-Gemisches, bevorzugt kleiner 15 Ma.-%, besonders bevorzugt kleiner 10 Ma.-%.

Vorteilhaft gelingt es so, in Schritt a) der Variante V1 ein Kohlenwasserstoffgemisch mit einem Massenanteil an Olefinen von weniger als 20 % der Gesamtmasse des gebildeten Kohlenwasserstoffgemischs zu generieren.

Die Anwesenheit eines höheren Wasseranteils (in Form von Wasserdampf) im Reaktor würde einerseits die Bildung niederer Olefine fördern und andererseits die Bildung schwerer Kohlenwasserstoffe reduzieren.

Weiterhin führt die Anwesenheit von Wasser auch zu einer gewissen Verkürzung der Katalysatorlebensdauer durch Desaktivierung (teilweise irreversibel) der aktiven sauren Zentren.

Die Kontrolle des Wassergehalts des Einsatzgases in den Reaktor ist dadurch nicht unbedeutend und ein geringer Wassergehalt im Einsatzalkohol von weniger als 20 % ist nicht nur für das gebildete Produkt, sondern auch wirtschaftlich von Vorteil.

Vorteilhaft ist damit der Anteil an Olefinen auch im Endprodukt geringer. Ein Benzin mit einem geringen Olefingehalt zeichnet sich durch eine sehr hohe Lagerstabilität aus. Insbesondere die Oktanzahl des Benzins bleibt über eine lange Zeit konstant.

In einer Ausführungsform ist der Alkohol aus nachwachsenden Rohstoffen hergestellt, womit nachhaltiges, CO₂-neutrales Benzin erzeugt werden kann.

Zur katalytischen Umsetzung wird der Alkohol, beispielsweise Methanol, in eine Reaktionszone im Reaktor, enthaltend einen Katalysator, eingespeist und dort vollständig oder unvollständig umgesetzt.

In einer Ausführungsform erfolgt die Einspeisung bei einem Druck zwischen 5 und 15 bar.

Die Eintrittstemperaturen in den Reaktor liegen in der Ausführungsform zur Erzeugung eines Produktbenzins mit niedrigem Aromatengehalt zwischen 300 und 350 °C.

Üblicherweise enthält der Katalysator Zeolithe als katalytisch wirksame Komponente. In einer Ausführungsform enthält der Katalysator ZSM-5-Zeolithe. In einer Ausführungsform wird ein ZSM-5-Katalysator mit einem SiO₂ zu Al₂O₃-Verhältnis des Zeolithen von mindestens 30:1, bevorzugt höher, verwendet.

Die Reaktion von Methanol zu Kohlenwasserstoffen an einem zeolithhaltigen Katalysator ist stark exotherm. Die Reaktionstemperatur hat einen sehr großen Einfluss auf die Reaktionsgeschwindigkeit und somit die Zusammensetzung des Reaktionsproduktes und die Selektivität des Prozesses. Steigen die Temperaturen aufgrund der Exothermie zu stark an, kann das zu einer schnellen Desaktivierung der aktiven Zentren des Katalysators führen, was sich negativ auf die Reaktionsdauer zwischen den Regenerierungen und die Standzeit des Katalysators auswirkt.

Voraussetzung für eine zielgerichtete Steuerung des Prozesses (hinsichtlich Reaktionsproduktzusammensetzung und Selektivität des Hauptproduktes) und eine ökonomische Prozessführung (Standzeit des Katalysators, Häufigkeit der Regenerierungen, Katalysatorbelastung) ist die Beherrschung der Exothermie der Reaktion durch interne Kühlung des Katalysatorbettes.

Nach dem Befüllen des Reaktors mit frischem Katalysator bzw. nach einer Katalysatorregenerierung trifft zu Beginn des Verfahrens der gesamte Einsatzalkohol auf die erste Katalysatorschicht auf und reagiert, was einen starken Temperaturanstieg in diesem Bereich zur Folge hat und eine Abschwächung der aktiven Zentren durch deren teilweise Desaktivierung. Der Anstieg der Temperatur in den nachfolgenden Katalysatorbereichen verringert sich allmählich, da von dem eingesetzten Alkohol immer weniger verbleibt und sich somit die umgesetzte Menge allmählich verringert.

Bei der nächsten Alkoholeinspeisung steigt die Temperatur im ersten Katalysatorbereich nicht mehr so stark an, da durch die bereits aufgetretenen hohen Temperaturen dieser Katalysatorbereich in seiner Aktivität abgeschwächt wurde. Die Temperaturfront wandert deshalb weiter in die nächste, noch aktivere Katalysatorschicht.

Eine effiziente Abfuhr der Reaktionswärme ist essentiell.

In einer Ausführungsform des Verfahrens, zur Erzeugung eines Produkts mit niedrigem Aromatengehalt, beträgt die Temperaturdifferenz im Katalysatorbett im Bereich der Temperaturfront max. 40 K zu Beginn einer Reaktionsperiode bis max. 20 K zum Ende einer Reaktionsperiode.

Erfindungsgemäß, zur Herstellung eines Produkts mit niedrigem Aromatengehalt, beträgt die Temperatur im Katalysatorbett, in Abhängigkeit vom Desaktivierungsgrad des Katalysators, zwischen 300 und 370 °C.

Die Abfuhr der Reaktionswärme aus dem Reaktionsbereich des Reaktors erfolgt in einer Ausführungsform durch Wärmeaustausch.

In einer Ausführungsform enthält der Reaktor innere Einbauten in Form von Platten, Rohren und anderen Elementen zum Wärmeaustausch, wobei sich der Katalysator in den Elementen und das Kühlmedium um die Elemente befindet oder umgekehrt. Die Strömung des Reaktionsgases kann in axialer Richtung (von oben nach unten oder von unten nach oben) oder in radialer Richtung (von einem inneren Zentralrohr nach außen) zur Vermeidung zu hoher Druckverluste erfolgen.

In den Wärmeaustauschelementen oder auch außerhalb dieser Elemente befindet sich der Wärmeträger. Als Wärmeträger kommen alle geeigneten Medien infrage, die in dem Temperaturbereich von Reaktion und Regenerierung verdampfen bzw. kondensieren oder den Aggregatzustand beibehalten und die Reaktionswärme durch Konvektion abführen.

Der Wärmeträger zur Abführung der Reaktionswärme durchläuft einen ständigen Kreislauf. Er nimmt die Wärme aus der Reaktionszone auf, strömt nach außen (außerhalb des Katalysatorbettes), gibt diese an einen zweiten Wärmeträger, bevorzugt Siedewasser, entweder im Inneren des Reaktors oder außerhalb des Reaktors in einem separaten Wärmetauscher ab und kehrt dann wieder in die Reaktionszone zur Wärmeaufnahme zurück.

Die Abkühlung des Wärmeträgers erfolgt außerhalb des Reaktors in einem Wärmetauscher, in welchem bevorzugt Sattdampf eines Druckes von mindestens 20 bar, besonders bevorzugt von mindestens 40 bar, erzeugt wird.

Der Wärmeträger kann dabei auch in mehreren Zonen mit jeweils unterschiedlicher Temperatur zur Reaktionszone geführt werden. Damit erreicht man, dass im Bereich hoher Reaktionstemperatur die Temperatur des Wärmeträgers niedriger eingestellt werden kann als in Bereichen mit niedrigerer Reaktionstemperatur. Die Anzahl der Wärmeträgerzonen mit jeweils unterschiedlicher Temperatur ist nach oben nur durch die Wirtschaftlichkeit des Reaktors begrenzt.

Als Reaktor kann auch ein Reaktor, in welchem die Reaktionswärme nach dem Prinzip von Wärmerohren abgeführt wird, verwendet werden. Bei diesem Prinzip befindet sich der Wärmeträger im Inneren von Wärmeaustauschelementen, wo er durch die Aufnahme der Wärme aus dem Reaktionssystem verdampft und durch Wärmeabgabe an einen zweiten Wärmeträger (bevorzugt Siedewasser), der sich bevorzugt im oberen Teil des Reaktors außerhalb des Katalysatorbettes befindet und die Wärmeaustauschelemente kühlt, wieder kondensiert. Kondensation und Verdampfung im Inneren der Wärmeaustauschelemente finden in einem geschlossenen System statt. Das Siedewasser verdampft bei Aufnahme der Wärme vom Wärmeträger. Der dabei entstehende Sattdampf hat bevorzugt einen Druck von mindestens 20 bar, besonders bevorzugt von mindestens 40 bar. Das Reaktionsgas kann in dieser Art von Reaktor entweder axial (von oben nach unten oder von unten nach oben) durch das Katalysatorbett strömen oder in radialer Richtung zur Vermeidung zu hoher Druckverluste (von einem inneren Zentralrohr nach außen).

Erfindungsgemäß ist der Reaktor **(R)** ein isothermer Rohrreaktor.

Erfindungsgemäß erfolgt in Schritt **I)** bzw. a) in V1 die Umsetzung des Alkohols, beispielsweise des Methanols, in ein Produktgemisch, enthaltend Wasser und ein Kohlenwasserstoffgemisch, enthaltend Olefine, n-Paraffine, iso-Paraffine, Aromaten und Naphthene.

Vorteilhaft beträgt der Massenanteil der Olefine im gebildeten Kohlenwasserstoffgemisch weniger als 20%, bevorzugt weniger als 10 %, besonders bevorzugt weniger als 5 %, insbesondere weniger als 2 % der Gesamtmasse des gebildeten Kohlenwasserstoffgemischs.

In einer Ausführungsform wird das Reaktionsgemisch anschließend in Wärmeaustauschern, Luftkühlern und Kühlwasserkühlern bis auf 45 °C, bevorzugt bis auf 35 °C abgekühlt. In einer Ausführungsform erfolgt eine Abkühlung unter Verwendung eines weiteren Kältemittelkühlers bis auf 5 °C.

Während der Abkühlung kondensieren Wasser, Alkohol und die kondensierbaren Kohlenwasserstoffe. Dieses Produktgemisch wird im nächsten Schritt in drei Fraktionen aufgetrennt.

Erfindungsgemäß erfolgt in Schritt **II**) bzw. b) in V1 die Auftrennung des in Schritt **I)** bzw. a) in V1 erhaltenen Produktgemischs in
- eine flüssige Kohlenwasserstoffphase,
- eine wässrige Phase, enthaltend den nicht umgesetzten Alkohol,
   und
- eine Gasphase, enthaltend C₁- bis C₅- Kohlenwasserstoffe.

In einer Ausführungsform erfolgt die Auftrennung des Produktgemischs in einem Dreiphasenseparator **(S).**

In der wässrigen Phase aus Schritt **II**) bzw. b) in V1 sind die während der Reaktion nicht umgesetzten Alkohole, beispielsweise Methanol, enthalten.

Nicht umgesetzte Zwischenprodukte können nach der Auftrennung in Schritt **II**) bzw. b) in V1 sowohl in der Gasphase (z. B. Dimethylether, Diethylether, Ethylen) als auch in der flüssigen Kohlenwasserstoffphase (z. B. Dimethylether, Diethylether, Dipropylether, Propylen, Butylen) enthalten sein.

In einer Ausführungsform wird der nicht umgesetzte Alkohol aus der wässrigen Phase abgetrennt. In der Variante V1 ist dies der Schritt c).

In einer Ausführungsform wird die in Schritt **II**) bzw. b) in V1 erhaltene wässrige Phase in einer Trenneinrichtung in Wasser und nicht umgesetzten Alkohol getrennt wird und der nicht umgesetzte Alkohol zu Schritt **I**) bzw. a) in V1 zurückgeführt.

In einer Ausführungsform erfolgt die Abtrennung in einer Trennvorrichtung **K3.** In einer Ausführungsform ist die Trennvorrichtung **K3** eine Kolonne.

In einer Ausführungsform erfolgt die Abtrennung bei 2 - 3 bar. Die Trennvorrichtung **K3** ist mit einem Kopfproduktkühler und einem Sumpferhitzer ausgestattet. In einer Ausführungsform beträgt die Kopftemperatur 70 bis 90 °C, bevorzugt 80 - 85 °C und die Sumpftemperatur 110 bis 150 °C, bevorzugt 120 - 130 °C.

In einer Ausführungsform wird der abgetrennte Alkohol als Einsatzstoff in den Reaktor zurückgeführt. In der Variante V1 ist dies der Schritt d).

In einer Ausführungsform wird er dazu flüssig, zusammen mit dem Einsatzalkohol, in den Gaskreislauf auf der Druckseite des Kompressors eingespeist und zusammen mit dem Kreislaufgas (d. h. mit der in Schritt **II**) bzw. b) in V1 gewonnenen Gasphase) in Wärmeaustauschern durch Abkühlung des heißen Reaktionsproduktes im Gegenstrom aufgeheizt und dabei verdampft. Nach einer Endaufheizung auf Reaktionstemperatur tritt das Gemisch gasförmig in den Reaktor ein.

Da es sich bei dem verwendeten Alkohol bevorzugt um Methanol handelt, wird dieser Kreislauf auch Methanolkreislauf genannt.

Erfindungsgemäß wird die in Schritt **II**) bzw. b) in V1 erhaltene Gasphase zu Schritt **I)** bzw. a) in V1, zurückgeführt.

In einer Ausführungsform enthält die Gasphase aus Schritt **II**) bzw. b) in Variante V1 leichte gasförmige Kohlenwasserstoffe, die nicht bei der Abkühlung des Reaktionsproduktes kondensieren. Das sind sowohl Inertkomponenten, die sich in der Reaktionszone des Reaktors nicht mehr umsetzen (wie z. B. Methan, Ethan, CO₂, Stickstoff) als auch Zwischenreaktionsprodukte (wie z. B. Dimethylether, Ethylen), die bei nochmaligem Einsatz im Reaktor weiter reagieren und zu den gewünschten Benzinkohlenwasserstoffen umgesetzt werden.

In einer Ausführungsform wird ein kleiner Anteil der Gasphase aus Schritt **II**) aus dem Gaskreislauf ausgespeist, um so die Anreicherung von Inertkomponenten im Gaskreislauf zu vermeiden.

Zweckmäßigerweise sollte ein vorgegebener Solldruck des Einsatzgases am Reaktoreintritt gehalten werden. Vorteilhaft kann die Druckeinstellung am Eintritt in den Reaktor über die Regelung der Ausspeisung von Gas aus dem Gaskreislauf bei einem Druckanstieg am Reaktoreintritt reguliert werden.

Der Hauptteil des Kreislaufgases wird zum Reaktor zurückgeführt.

In einer Ausführungsform wird die in Schritt **II**) bzw. b) in Variante V1 erhaltene Gasphase geteilt und der erste Teil (=Hauptteil) zum Reaktor **R** zurückgegeben. Die Teilung erfolgt durch Öffnung eines Ventils in der Gasleitung vom Dreiphasenseparator zur Trennvorrichtung **K4,** welches bei Überdruck am Reaktoreingang öffnet. Der andere, zweite Teil der Gasphase strömt zum Kompressor, welcher das Gas verdichtet und zum Reaktor befördert. In der Variante V1 ist dies der Schritt e). In einer Ausführungsform ist die Trennvorrichtung **K4** eine Kolonne.

Der Hauptteil der Gasphase (erster Teil) wird mit Hilfe eines Kompressors als Kreislaufgas zum Reaktoreingang (zum Schritt **I)** bzw. a) in V1) zurück gefördert und bewirkt dort eine Verdünnung des Einsatzstoffes. Mittels des Kompressors werden die Druckverluste im Gaskreislauf ausgeglichen und es kann die Kreislaufgasmenge (entsprechend dem Sollvolumenstrom) eingestellt werden.

Der andere Teil der Gasphase (zweiter Teil) wird durch Regelung des Druckes am Eintritt des Reaktors aus der Anlage ausgeschleust.

Der zweite Teil der Gasphase wird in einem weiteren Schritt aufgetrennt. In der Variante V1 ist dies der Schritt **f).**

In einer Ausführungsform wird die Teilung der Gasphase nicht durchgeführt und damit kein Kreislaufgasstrom (d. h. Teil der Gasphase aus Schritt **II**) bzw. b) in V1) zum Reaktor zurückgeführt.

In einer Ausführungsform erfolgt die Auftrennung des zweiten Teils der Gasphase in einer Trennvorrichtung **K4,** beispielsweise in einer Separationsgaskolonne. Die Trennung der Gasphase erfolgt bei einem Druck von 10 bis 20 bar, bevorzugt bei 15 - 16 bar. Es ist erforderlich, das Gas vor Eintritt in die Trennvorrichtung auf diesen Druck zu komprimieren. Die Separationsgaskolonne ist mit einem Kopfproduktkühler ausgestattet. Die Kopftemperatur beträgt -30 bis -10°C, bevorzugt -20 °C bis -25 °C.

Dabei erfolgt die Abtrennung von C₃- bis Cs-Kohlenwasserstoffen.

Diese abgetrennten C₃- bis Cs-Kohlenwasserstoffe werden in einem nachfolgenden Schritt mit der in Schritt **II**) bzw. b) in V1 erhaltenen flüssigen Kohlenwasserstoffphase vereint. In der Variante V1 ist dies der Schritt g).

In einer Ausführungsform wird von einem Teil der in Schritt **II**) bzw. b) in V1 erhaltenen Gasphase in einer Trenneinrichtung eine C₃- bis C₅- Kohlenwasserstofffraktion abgetrennt und diese mit der in Schritt **II**) erhaltenen flüssigen Kohlenwasserstoffphase vereint.

In einer Ausführungsform wird diese Kohlenwasserstoffphase dann in Schritt **IV)** in eine C₃-bis C₄-Fraktion und eine Benzinkohlenwasserstofffraktion, enthaltend eine durolhaltige schwere Aromatenfraktion, getrennt.

Die in Schritt **II**) bzw. b) in Variante V1 gewonnene flüssige Kohlenwasserstoffphase enthält, in Abhängigkeit von den Reaktionsbedingungen, mehr oder weniger C₃- und C₄-Kohlenwasserstoffe. Bei unvollständigem Methanolumsatz ist auch das Zwischenprodukt Dimethylether (DME) Bestandteil des Reaktionsproduktes. Ein Teil des DME's ist in der flüssigen Kohlenwasserstoffphase enthalten. Hauptsächlich sind C₅₊-Verbindungen in der flüssigen Kohlenwasserstoffphase enthalten.

C₃- und C₄-Kohlenwasserstoffe können die Paraffine Propan, Butan und iso-Butan sein. Bei bestimmten Reaktionsbedingungen, bei denen sich der Einsatzstoff unvollständig umsetzt, können auch olefinische Zwischenprodukte, wie Propylen, Butylen, iso-Butylen, aus dem Reaktor austreten und in der flüssigen Kohlenwasserstoffphase enthalten sein.

In einer Ausführungsform wird die im Schritt **II**) bzw. b) in Variante V1 erhaltene flüssige Kohlenwasserstoffphase zusammen mit der erhaltenen C₃- bis C₅- Fraktion in eine Trennvorrichtung **K1** eingespeist.

Eine schwere Aromatenfraktion im Sinne der Erfindung bedeutet eine Fraktion, enthaltend höhermolekulare aromatische Kohlenwasserstoffe. Dies sind aromatische Kohlenwasserstoffverbindungen, enthaltend 9 bis 11 Kohlenstoffatome. Der Benzolring kann dabei mehrfach methyliert sein.

In einer Ausführungsform wird aus der flüssigen Kohlenwasserstoffphase die C₃- bis C₄-Fraktion als Kopfprodukt in einer Trennvorrichtung **K1,** die eine Kolonne sein kann, abgetrennt. Die Trennung in der Kolonne erfolgt bei einem Druck von 10 bis 20 bar, bevorzugt 13 bis 17 bar. Die Kolonne ist mit einem Kopfproduktkühler und einem Sumpferhitzer ausgestattet. Die Kopftemperatur beträgt 10 bis 90 °C, bevorzugt 20 - 70 °C und die Sumpftemperatur 150 bis 250 °C, bevorzugt 180 - 225 °C. Die Parameter hängen stark von der Zusammensetzung des aufzutrennenden Gemisches ab.

In einer Ausführungsform besteht das Kopfprodukt aus den Paraffinen Propan, Butan und iso-Butan. Mindestens ein Teil dieser C₃- bis C₄-Fraktion einer solchen Zusammensetzung kann als verkaufsfähiges Produkt "Flüssiggas (LPG)" abgezogen werden.

In einer Ausführungsform enthält die C₃- bis C₄-Fraktion Olefine und/oder Dimethylether und/oder gesättigte C₃- bis C₄-Kohlenwasserstoffe.

In einer Ausführungsform wird mindestens ein Teil der in Schritt **IV**) abgetrennten C₃- bis C₄-Kohlenwasserstofffraktion im Kreislauf zum Eintritt des Reaktors als Einsatzstoff zu Schritt **I)** bzw. a) in Variante V1 zurückgeführt.

Die in der Trennvorrichtung abgetrennte Benzinkohlenwasserstofffraktion (C₅₊-Fraktion) enthält erfindungsgemäß eine Stabilbenzinfraktion und eine durolhaltige schwere Aromatenfraktion, enthaltend Durol, iso-Durol und andere mehrfach methylierte Aromaten.

Durol (1,2,4,5-Tetramethylbenzol) und iso-Durol (1,2,3,5-Tetramethylbenzol) sind schwere methylierte Aromaten, die die Eigenschaft haben, im Temperaturbereich der Anwendung von Benzin (Umgebungstemperatur, bevor der Kraftstoff in den Motor eintritt) zu erstarren. So beträgt die Erstarrungstemperatur von Durol 79,2 °C und von iso-Durol -20 °C. Aus diesem Grund sollten diese Tetramethylbenzole nicht im Benzin enthalten sein.

Im erfindungsgemäßen Verfahren werden sie, beispielsweise durch Destillation, aus dem flüssigen Anteil des Kohlenwasserstoffproduktes abgetrennt.

Die Benzinkohlenwasserstofffraktion aus Schritt **IV)** bzw. h) in Variante V1, enthaltend C₅₊-Kohlenwasserstoffe, wird im Schritt **V)** bzw. j) in V1 weiter in eine durolhaltige schwere Aromatenfraktion und eine Stabilbenzinfraktion aufgetrennt.

In einer Ausführungsform enthält die durolhaltige schwere Aromatenfraktion Durol, iso-Durol und mehrfach methylierte Aromaten.

In einer Ausführungsform erfolgt die Auftrennung der Benzinkohlenwasserstofffraktion in einer Trennvorrichtung **K2.** Die Trennvorrichtung **K2** kann eine Kolonne sein, die mit einem Kopfproduktkühler und einem Sumpferhitzer ausgestattet ist.

In einer Ausführungsform erfolgt die Trennung bei einem Druck von 1 bis 3 bar, bevorzugt 1,2 bis 2,5 bar, einer Kopftemperatur von 50 bis 90 °C, bevorzugt 60 - 75 °C und einer Sumpftemperatur im Bereich von 200 bis 260 °C, bevorzugt 230 - 240 °C.

Dabei werden eine Stabilbenzinfraktion und eine durolhaltige schwere Aromatenfraktion erhalten. Die Stabilbenzinfraktion ist das gewünschte Produktbenzin mit einer hohen Oktanzahl von > 90 ROZ, bevorzugt >/= 95 ROZ.

In einer Ausführungsform wird Durol aus der durolhaltigen schweren Aromatenfraktion auskristallisiert.

In einer Ausführungsform wird dazu die schwere Aromatenfraktion in einen Kristallisator **KR1** gegeben, in welchem durch Kristallisation das bei ca. 79 - 80 °C auskristallisierende Durol abgetrennt wird.

In einer Ausführungsform wird das auskristallisierte Durol in Alkohol und/oder in Benzinkohlenwasserstoffen gelöst und zusammen mit den Einsatz- und rückgeführten Alkoholen zum Eintritt des Reaktors zurückgeführt.

In einer Ausführungsform wird nicht reines Durol, sondern das Sumpfprodukt der Trennvorrichtung **K2** (schwere durolhaltige Aromatenfraktion) ohne Abtrennung des Durols ganz oder teilweise zum Reaktor zurückgeführt. In diesem Fall sind kein Kristallisator **KR1** und keine Vorrichtung **V1** zum Lösen des Durols erforderlich.

Vorteilhaft ist das erfindungsgemäße Verfahren dadurch charakterisiert, dass durch die Verknüpfung mehrerer Stoffkreisläufe zum / vom Reaktor während der Synthese von Kohlenwasserstoffen die Einstellung von variablen Reaktionsbedingungen in breiten Bereichen ermöglicht wird.

Besonderes Merkmal des Verfahrens ist die hohe Flexibilität der Produktzusammensetzung durch Variation der Prozessparameter. Das Verfahren soll ermöglichen, auf spezielle Anforderungen an die Benzinzusammensetzung zu reagieren. So kann ein Benzin oder ein Benzinblend einer Zusammensetzung entsprechend wirtschaftlichen und ökologischpolitischen Erfordernissen erzeugt werden. Insbesondere ermöglicht das Verfahren, ein bei der Verbrennung emissionsarmes Benzin mit reduziertem CO₂-Ausstoß und verringertem Partikelbildungspotenzial zu erzeugen. Nach Bedarf kann ein Benzinblend mit hoher Oktanzahl erzeugt werden, welches durch einen hohen Anteil an hochoktanigen isoparaffinischen Komponenten charakterisiert wird.

Für Hochleistungsmotoren sind Benzine mit sehr hoher Oktanzahl (ROZ 98 und höher) erforderlich. Sehr hohe Oktanzahlen können nicht mehr allein durch einen hohen iso-Paraffingehalt des Benzins erreicht werden. Dazu ist zusätzlich zu den iso-Paraffinen noch ein gewisser Anteil an hochoktanigen Aromaten im Benzin notwendig. Aromaten können Oktanzahlen bis zu ca. ROZ 120 bis 150 erreichen (z. B. Toluol - ROZ 124; p-Xylol - ROZ 146).

Aufgrund des höheren Aromatengehalts kann ein Premiumbenzin nicht das ökologisch vorteilhafte hohe H:C-Verhältnis erreichen, hat jedoch sehr gute Verbrennungseigenschaften durch die hohe Oktanzahl, wodurch der Kraftstoffverbrauch verringert wird. Trotz des höheren Kohlenstoffgehalts kann auch dieses Benzin einen Beitrag zur Minderung der CO₂-Emissionen leisten, indem es in einem PtL-Prozess ("power to liquid") erzeugt wird und möglichst aus Rohstoffen, die aus "nachwachsender" (Biomasse zur CO₂- Erzeugung) oder "regenerativer" (Wasser zur Wasserstofferzeugung) Quelle oder aus Industrieabgasen (CO₂ bzw. CO₂ und H₂) stammen.

Vorteilhaft kann das erfindungsgemäße Verfahren und die erfindungsgemäße Anlage nicht nur in die PtL-Prozesskette eingebunden werden, sondern auch mit konventionellen Prozessen der Erzeugung von Synthesegas aus fossiler Quelle (z. B. aus Erdgas) und anschließender Methanolsynthese aus dem Synthesegas kombiniert werden.

In dem erfindungsgemäßen Verfahren kann vorteilhaft stabilisiertes Rohmethanol, ohne einer Notwendigkeit der weiteren Aufbereitung des Rohmethanols durch eine kostenintensive Abtrennung des darin enthaltenen Wassers sowie anderer Alkohole und Oxygenate, als Einsatzstoff verwendet werden.

Die erfindungsgemäß hergestellten Benzine können nicht nur direkt zum Einsatz im Motor, sondern auch als Blendbenzine in Raffinerien zur Zumischung in den Benzinpool verwendet werden. Als Blendbenzine dienen sie zum einen zur Erhöhung der Oktanzahl und zum anderen zur Verminderung des CO₂-footprints des Raffineriebenzins.

Bei Verwendung eines Benzins mit hohem iso-Paraffingehalt und niedrigem Aromatengehalt als Blendbenzin wird der Kohlenstoffanteil im gemischten Fertigbenzin reduziert und auf diese Weise der CO₂-Gehalt im Abgas und das Partikelbildungspotenzial bei der Verbrennung.

Vorteilhaft können mit dem erfindungsgemäßen Verfahren Benzine mit variabler Zusammensetzung erzeugt werden, indem Prozessparameter angepasst werden.

Im Sinne der Erfindung enthält ein Benzin mit hohem iso-Paraffingehalt und niedrigem Aromatengehalt (aromatenarm) 50 - 65 Ma.-% iso-Paraffine und 20 - 35 Ma.-% Aromaten.

In einer Ausführungsform enthält ein Benzin mit hohem iso-Paraffingehalt und niedrigem Aromatengehalt (aromatenarm) 50 - 60 Ma.-% iso-Paraffine und 25 - 35 Ma.-% Aromaten.

Ma.-% bedeutet der Massenanteil an der Gesamtmasse in %.

Prozessparameter, die individuell eingestellt werden können, sind beispielsweise Katalysatorbelastung, Kontaktzeit mit dem Katalysator, Reaktionstemperatur, Strömungsgeschwindigkeit, Gesamtdruck im Reaktor und die Partialdrücke der einzelnen Komponenten.

Die Katalysatorbelastung wird über die Kenngröße der Raumgeschwindigkeit (LHSV - *liquid hourly space velocity*) angegeben. Diese ergibt sich aus dem Quotienten von Flüssigkeitsvolumenstrom des eingesetzten Alkohols und Katalysatorvolumen.

Im erfindungsgemäßen Verfahren ist die LHSV definiert als flüssige Alkoholmenge (bei Raumtemperatur und Normaldruck), bezogen auf 1 m³ Katalysator, in m³_{Alkohol}/h m³_{Kat}.

Die Belastung des Katalysators gibt die Menge an reinem Einsatzalkohol (ohne Wasser) pro Stunde und Katalysatorvolumen an.

Entsprechend dem Reaktionsmechanismus der oben beschriebenen MTH-Reaktion entstehen bei der Umsetzung von Methanol zunächst DME und Olefine. Diese ersten Reaktionsschritte erfolgen relativ langsam, während die nachfolgenden Schritte der Reaktion der Olefine mit dem Methanol zu weiteren Olefinen bzw. CH₂-Fragmenten und die Folgereaktionen sehr schnelle Reaktionen sind.

### Erzeugung von Benzin mit hohem iso-Paraffingehalt/ niedrigem Aromatengehalt (aromatenarmes, iso-Paraffin-reiches Benzin)

Entscheidend für die Bildung eines hohen Anteils an iso-Paraffinen im Produktgemisch ist zunächst die unvollständige Umsetzung der Einsatzstoffe, beispielsweise des Methanols. Die nachfolgende Erläuterung wird am Beispiel des Einsatzes von Methanol gezeigt.

Eine hohe Belastung des Katalysators mit Methanol führt zu einer unvollständigen Reaktion bzw. nur zu einem Teilumsatz des in den Reaktor eingespeisten Methanols. Aus dem Reaktor treten nicht umgesetztes Methanol sowie Zwischenprodukte, wie Dimethylether und Olefine, aus. Mit der Erhöhung der Belastung sinkt der Aromatengehalt im Produkt. Eine Rückführung der olefinhaltigen C₃ - C₄ - Fraktion fördert die Bildung von iso-Paraffinen während der Reaktion.

In einer Ausführungsform beträgt die Alkoholbelastung (beispielsweise Methanolbelastung) des Katalysators zur Erzeugung eines Benzins mit hohem iso-Paraffingehalt zwischen 2 und 5 m³_{MeoH}/h m³_{Kat}., bevorzugt zwischen 2,5 und 4 m³_{MeoH}/h m³_{Kat}., bezogen auf das im Reaktor enthaltene Gesamtvolumen an Katalysator.

Die hohe Belastung des Katalysators mit Methanol bedingt eine Erhöhung des Anteils an Olefinkohlenwasserstoffen im Reaktionsprodukt.

Auch die Kontaktzeit der Einsatzstoffe im Katalysatorbett beeinflusst die Produktzusammensetzung. Vorteilhaft kann im erfindungsgemäßen Verfahren die Kontaktzeit im Katalysatorbett in Strömungsrichtung des Reaktionsgases an die Erfordernisse der beschriebenen Teilschritte der MTH-Reaktion angepasst werden.

In einer Ausführungsform erfolgt die Einstellung von Belastung und Kontaktzeit des Katalysators durch eine Änderung des Strömungsquerschnittes durch konstruktive Maßnahmen, beispielsweise durch Änderung des Durchmessers der Katalysatorrohre. Eine Vergrößerung des Strömungsquerschnittes im Katalysatorbett bewirkt eine Erhöhung der Kontaktzeit und eine Verringerung der Katalysatorbelastung. Durch Verengung des Strömungsquerschnittes wird der gegenteilige Effekt bewirkt.

Erfindungsgemäß ist zur Erzeugung eines Benzins mit hohem iso-Paraffingehalt der Katalysator partiell mit Inertmaterial verdünnt. Durch das Zumischen von Inertmaterial erfolgt eine gezielte Einflussnahme auf das Katalysatorvolumen. Durch die Verdünnung des Katalysators mit nichtaktivem Material wird das Katalysatorvolumen in dem betroffenen Reaktorabschnitt verkleinert, die Strömungsverhältnisse bleiben jedoch gleich (vorausgesetzt, das Inertmaterial hat die gleiche Teilchenform wie der Katalysator und das Lückenvolumen entspricht dem der Katalysatorschüttung ohne Inertmaterial). Die Kontaktzeit wird verringert und die Belastung des Katalysators erhöht. Beim Einsatz von Zeolithen als Katalysator werden, in einer Ausführungsform, Aluminiumoxidmaterialien (Keramik) als Inertmaterial verwendet.

Im Eintrittsbereich des Methanols in das Katalysatorbett finden die langsamen Reaktionen der Dehydratisierung mit Bildung von Dimethylether und Wasser sowie der Bildung von Olefinen bzw. CH₂-Fragmenten aus den Oxygenaten Methanol und DME statt.

Zur Erzeugung von Benzin mit einem hohen iso-Paraffin-Gehalt werden im ersten Abschnitt des Katalysatorbettes in Strömungsrichtung Belastungen und Kontaktzeiten eingestellt, die den unvollständigen Umsatz des Methanols zu DME gewährleisten.

Im folgenden Abschnitt des Katalysatorbetts in Strömungsrichtung sind sehr kurze Kontaktzeiten und sehr hohe Katalysatorbelastungen erforderlich, um zu gewährleisten, dass die MTH-Reaktion bei der Olefinbildung abgebrochen wird.

Erst im letzten, unteren Abschnitt des Katalysatorbettes müssen längere Kontaktzeiten und geringere Katalysatorbelastungen eingestellt werden, damit sich die gewünschten Benzinkohlenwasserstoffe mit einem geringen Aromatengehalt bilden.

Dabei ist es vorteilhaft, wenn in diesem Bereich die Belastung so hoch ist, dass die Zwischenprodukte nicht bis zu den Endprodukten weiterreagieren können und im Reaktionsprodukt noch nicht umgesetzte Komponenten - wie Methanol, DME und Olefine - enthalten sind.

Erfindungsgemäß erfolgt eine Rückführung der in Schritt **IV)** bzw. h) in Variante V1 abgetrennten C₃- bis C₄-Fraktion zum Reaktoreigang als Einsatzstoff zu Schritt **I)** bzw. a) in Variante V1. Die Bildung von iso-Paraffinen wird dadurch angeregt. Am Eintritt in das Katalysatorbett können damit sofort die Olefinkohlenwasserstoffe bzw. die daraus entstehenden CH₂-Fragmente mit dem Alkohol, beispielsweise Methanol, reagieren und somit zu einer Beschleunigung der MTH (Methanol to Hydrocarbons) - Reaktion führen. Im weiteren Reaktionsverlauf methylieren die CH₂-Fragmente die anderen entstandenen Kohlenwasserstoffe - wie n-Paraffine, Naphthene und Aromaten. Da die Aromatenbildung bei den oben beschriebenen Reaktionsbedingungen gehemmt wird, entstehen hauptsächlich iso-Paraffine durch Methylgruppenanlagerung an die gebildeten n-Paraffine.

In einer Ausführungsform wird zur Verstärkung des Verzweigungsgrades der iso-Paraffine im letzten Abschnitt des Katalysatorbettes, beispielsweise im unteren Drittel, ein mit einer aktiven Komponente zur Aktivierung von Isomerisierungsreaktionen, wie beispielsweise eine metallische Komponente, z. B. Nickel, modifizierter Zeolithkatalysator vom Typ ZSM-5 eingesetzt.

Die Produktzusammensetzung kann vorteilhaft durch Variation des Umsatzgrades der Oxygenate, beispielsweise des Methanols, eingestellt werden.

Der Umsatzgrad von beispielsweise Methanol wird, wie oben bereits beschrieben, durch die Reaktionstemperatur und die Belastung des Katalysators eingestellt. Durch eine hohe Belastung (große Methanolmenge pro Katalysatorvolumen > 3 h⁻¹) des Katalysators mit Einsatzmethanol und bei niedrigen Reaktionstemperaturen (300 - 350 °C) kann erreicht werden, dass nicht das gesamte eingespeiste Methanol am Katalysator umgesetzt wird und ebenso die in der Reaktion entstehenden Benzinkohlenwasserstoffe nur teilweise bis zum Endprodukt der Aromaten weiterreagieren.

Neben dem nicht umgesetzten Methanol reagiert bei diesen Bedingungen auch das Zwischenprodukt Dimethylether, welches durch Dehydratation des Methanols entsteht, nur unvollständig zu Kohlenwasserstoffen weiter.

In einer Ausführungsform liegt der Umsatzgrad des Methanols zur Erzeugung eines iso-Paraffin- reichen Benzins zwischen 70 und 90 %, bevorzugt zwischen 70 und 80 %.

Ein weiterer Prozessparameter zur Einstellung der Produktzusammensetzung ist der Methanolpartialdruck.

Der Methanolpartialdruck beschreibt den Anteil des Methanols am Gesamteinsatzstoff in den Reaktor. Er ist definiert als Stoffmengenanteil des Methanols vom Einsatzgas multipliziert mit dem Gesamtdruck am Eintritt in den Reaktor. Der Methanolpartialdruck gibt den Verdünnungsgrad des Methanols mit Inertkomponenten wider und beeinflusst den Umsatzgrad des Methanols. Niedrige Methanol-Partialdrücke zeigen eine hohe Verdünnung des Eduktes an, der Methanolumsatzgrad steigt mit dem Grad der Verdünnung des Eduktes.

Durch die Kreislaufführung bestimmter Komponenten (beispielsweise: Kreislaufgas - C₁ bis C₄- Kohlenwasserstoffe sowie DME) oder Rückführung der C₃/C₄- Flüssiggaskomponenten wird ein bestimmter Volumenstrom durch das Katalysatorbett eingestellt, wodurch sich, in Abhängigkeit vom Anströmquerschnitt des Katalysatorbettes und von den Parametern Druck und Temperatur, eine bestimmte Strömungsgeschwindigkeit des Reaktionsgases im Reaktor einstellt.

Für eine gute Wärmeübertragung aus der Reaktionszone an den Wärmeträger ist eine hohe Strömungsgeschwindigkeit des Reaktionsgemisches durch das Katalysatorbett erforderlich. Zu niedrige Strömungsgeschwindigkeiten bedingen einen niedrigen gasseitigen Wärmeübergangskoeffizienten. Zu hohe Strömungsgeschwindigkeiten verursachen hohe Druckverluste über dem Katalysatorbett, insbesondere, wenn der Strömungsweg durch das Katalysatorbett mehrere Meter lang ist.

Ein mittlerer Bereich der Strömungsgeschwindigkeit, der sowohl einen guten Wärmeübergang als auch niedrige Druckverluste liefert, sollte deshalb Anwendung finden.

In einer Ausführungsform beträgt die Strömungsgeschwindigkeit des Reaktionsgemisches 0,8 bis 3,0 m/s, bevorzugt 1,5 bis 2,5 m/s.

Erfindungsgemäß beträgt der Stoffmengenanteil des Alkohols, beispielsweise des Methanols, an der Gesamtstoffmenge des Einsatzgases am Reaktoreintritt 25 bis 50 Mol.-%, bevorzugt 25 bis 40 Mol.-%, wobei das Einsatzgas den Einsatzalkohol und die rückgeführten Komponenten umfasst.

In einer Ausführungsform versteht man unter "rückgeführten Komponenten" die Kohlenwasserstofffraktionen, die in den einzelnen Verfahrensschritten als "zu Schritt I) zurückgeführt" bezeichnet werden.

Die rückgeführten Komponenten enthalten zumindest die in Schritt **II**) erhaltene Gasphase (Kreislaufgas) und die in Schritt IV) abgetrennte C₃- bis C₄-Fraktion.

In einer weiteren Ausführungsform enthalten die rückgeführten Komponenten zusätzlich Alkohol, der aus der in Schritt **II**) erhaltenen wässrigen Phase abgetrennt wurde.

Diese Ausführungsformen lassen sich beliebig miteinander kombinieren.

Zur Erzeugung eines Benzins mit hohem iso-Paraffingehalt enthalten die rückgeführten Komponenten bevorzugt auch einen Teil der in Schritt V) erhaltenen schweren durolhaltigen Aromatenfraktion.

Zur Erzeugung eines Benzins mit niedrigem Aromatengehalt und hohem iso-Paraffingehalt muss die Reaktionstemperatur im Katalysatorbett möglichst niedrig sein, jedoch oberhalb der Initiierungstemperatur der Reaktion liegen.

Es muss ein hoher Anstieg der Reaktionstemperatur durch die Exothermie der Reaktion vermieden werden. Insbesondere im Eintrittsbereich des Methanols in die Katalysatorschüttung müssen hohe Temperaturanstiege vermieden werden, was durch die Verdünnung des Katalysators mit Inertmaterial erreicht wird.

Erfindungsgemäß wird der Katalysator partiell mit Inertmaterial verdünnt.

Erfindungsgemäß wird der erste Katalysatorbereich, auf welchen zu Beginn der Reaktionsphase die größte Menge des Einsatzalkohols auftrifft (bevorzugt ca. 1/5 bis 1/6 der Schüttung), mit Inertmaterial eines Massenanteils von 5 bis 30 %, besonders bevorzugt von 10 bis 25 %, bezogen auf die Gesamtmasse an Katalysator und Inertmaterial, verdünnt, um hohe Temperaturanstiege zu vermeiden.

In einer Ausführungsform werden Aluminiumoxidmaterialien (Keramik) als Inertmaterial verwendet.

In einer Ausführungsform liegen die Reaktionstemperaturen zur Erzeugung eines Benzins mit niedrigem Aromatengehalt und hohem iso-Paraffingehalt zwischen 300 und 370 °C, bevorzugt zwischen 300 und 350 °C.

In einer Ausführungsform beträgt der Gesamtdruck im Reaktor zur Erzeugung eines Benzins mit niedrigem Aromatengehalt und hohem iso-Paraffingehalt < 15 bar, bevorzugt < 11 bar, besonders bevorzugt < 10 bar.

In einer Ausführungsform wird Durol aus der durolhaltigen schweren Aromatenfraktion auskristallisiert.

In einer Ausführungsform wird dazu die schwere Aromatenfraktion in einen Kristallisator **KR1** gegeben, in welchem durch Kristallisation das bei ca. 79 - 80 °C auskristallisierende Durol abgetrennt wird.

In einer Ausführungsform wird zur Erzeugung eines Benzins mit niedrigem Aromatengehalt und hohem iso-Paraffingehalt das auskristallisierte Durol im Einsatzalkohol oder im rückgeführten Alkohol aus der Trennvorrichtung **K3** und/oder in Benzinkohlenwasserstoffen, beispielsweise der Stabilbenzinfraktion aus Schritt **V),** gelöst. Die Durol-Lösung wird dann zu Schritt **I)** bzw. a) in Variante V1 in den Reaktor in einem Kreislauf zurückgeführt.

In einer Ausführungsform wird zur Erzeugung eines Benzins mit hohem iso-Paraffingehalt ein Teil der in Schritt **V)** erhaltenen schweren durolhaltigen Aromatenfraktion zu Schritt **I)** zurückgeführt.

Auf diese Weise kann die Aromatisierungsaktivität des Katalysators beeinflusst werden. Durch die Rückführung von Durol zum Reaktor kann die Bildung der anderen Aromaten verringert werden, so dass diese Methode zur Erzeugung eines Benzins mit niedrigem Aromatengehalt angewandt werden kann. Zusätzliche positive Effekte sind die Erhöhung der Zwischenregenerierdauer bzw. Verringerung der Häufigkeit notwendiger Katalysatorregenerierungen und die Minderung der Alterung des Katalysators und Erhöhung seiner Lebensdauer.

In einer Ausführungsform wird zur Erzeugung eines Produktbenzins mit einem hohen iso-Paraffingehalt und einem geringen Aromatengehalt zusätzlich Wasserstoff in den Methanolkreislauf und/oder in den Reaktor eingespeist.

Der Wasserstoff dient der Absättigung der wasserstoffarmen olefinischen und aromatischen Komponenten und deren Umwandlung zu n- und iso-Paraffinen und Naphthenen und der Vermeidung bzw. Verringerung der Bildung von Koks auf der Katalysatoroberfläche. Eine verminderte Koksbildung erhöht die Zwischenregenerierdauer und die gesamte Lebensdauer des Katalysators.

Die Zuspeisung von Wasserstoff ermöglicht die Erhöhung des iso-Paraffinanteils im Reaktionsprodukt. Durch die Erhöhung des iso-Paraffingehalts / Verringerung des Aromatengehalts erhöht sich das H/C-Verhältnis des Produktes, so dass ab einem bestimmten Verhältnis der iso-Paraffine zu den Aromaten im Produkt die stöchiometrisch erforderliche Wasserstoffmenge größer ist als die im Einsatzalkohol vorhandene.

Ausgehend von Methanol, als dem bevorzugten Einsatzstoff, kann durch die Zuspeisung von Wasserstoff ein maximaler iso-Paraffingehalt des Benzins von 75 Ma.-% (Masseprozent = Massenanteil an der Gesamtmasse in %) bei einem Aromatengehalt im Benzin von ca. 11 Ma.-% erreicht werden. Die erforderliche Wasserstoffmenge beträgt ca. 1 bis 5 Ma.-%, bevorzugt 1 bis 2 Ma.-%, bezogen auf den umzusetzenden Alkohol, beispielsweise Methanol.

Der Mindesteinspeisedruck des Wasserstoffs muss über dem Systemdruck im Gaskreislauf liegen.

In einer Ausführungsform wird der Wasserstoff mit einem Druck von 8 bis 15 bar, bevorzugt 9 bis 12 bar, besonders bevorzugt 10 bar eingespeist.

In einer Ausführungsform wird zur Verringerung der Aromatenbildung, und/oder zur Erzeugung eines Benzins mit hohem iso-Paraffingehalt, insbesondere zu Beginn eines Reaktionszyklusses, ein Teil der durolhaltigen schweren Aromatenfraktion, beispielsweise aus Schritt j) in Variante V1 ohne Abtrennung des Durols, zum Reaktor zu Schritt I) bzw. a) in V1 zurückgeführt. Durch diese Methode wird der Aufbau eines Carbonpools auf der aktiven Katalysatoroberfläche gefördert, wodurch seine Aktivität abgeschwächt wird und weniger Aromaten gebildet werden. Es wird die Alterung des Katalysators verringert und seine Lebensdauer erhöht.

Nach oben sind Reaktionstemperaturen begrenzt, um eine irreversible Desaktivierung des Katalysators zu vermeiden.

In einer Ausführungsform wird nur im Eintrittsbereich des Alkohols (ca. 20 bis 100 mm Katalysatorschütthöhe) der Katalysator mit 5 - 30 % Inertmaterial, bevorzugt 10 - 25 %, vermischt. Dies führt zur Vermeidung einer Katalysatordesaktivierung durch zu hohe Temperaturen, da in diesem Bereich die umgesetzte Alkoholmenge am größten ist.

### Anlage:

Gegenstand der Erfindung ist auch eine Anlage zur Synthese eines synthetischen Benzins mit hohem iso-Paraffingehalt im Bereich 50-65 Ma% und mit niedrigem Aromatengehalt im Bereich 20-35 Ma%, umfassend die Komponenten:
I. Isothermen Rohrreaktor **(R),** enthaltend einen Katalysator, zur katalytischen Umsetzung von Alkoholen in ein Produktgemisch, enthaltend ein Kohlenwasserstoffgemisch und Wasser,
   wobei der Katalysator im Eintrittsbereich des Alkohols partiell mit 5-30 Massen-% Inertmaterial, bezogen auf die Gesamtmasse an Katalysator und Inertmaterial, verdünnt ist,
II. Dreiphasenseparator **(S)** zur Auftrennung des im Rohrreaktor (R) erhaltenen Produktgemischs in eine flüssige Kohlenwasserstoffphase, eine wässrige Phase, enthaltend nicht umgesetzten Alkohol, und eine Gasphase,
III. eine erste Trennvorrichtung **(K1),** die geeignet ist, eine flüssige Kohlenwasserstoffphase, enthaltend Kohlenwasserstoffe mit 3 bis 11 Kohlenstoffatomen, in eine C₃-C₄- Fraktion und eine C₅₊- Fraktion aufzutrennen,
IV. eine zweite Trennvorrichtung **(K2),** die geeignet ist, eine Kohlenwasserstoffphase, enthaltend Kohlenwasserstoffe mit 5 bis 11 C-Atomen, in eine durolhaltige schwere Aromatenfraktion und eine Stabilbenzinfraktion zu trennen,
V. eine Verbindungsleitung **(VL1)** vom Rohrreaktor **R** zum Dreiphasenseparator **S** zum Transport des Reaktionsproduktes, enthaltend C₁-C₁₁-Kohlenwasserstoffe, Wasser und Alkohol,
VI. eine Verbindungsleitung **(VL3)** vom Dreiphasenseparator **(S)** zur ersten Trennvorrichtung **(K1),** die geeignet ist, eine flüssige Kohlenwasserstoffphase, enthaltend Kohlenwasserstoffe mit 3 bis 11 Kohlenstoffatomen, zu transportieren,
VII. eine Verbindungsleitung **(VL5)** von der ersten Trennvorrichtung **(K1)** zur zweiten Trennvorrichtung **(K2),** die geeignet ist, eine C₅₊-Kohlenwasserstofffraktion zu transportieren,
VIII. eine Rückführungsleitung **(RL1)** vom Dreiphasenseparator **(S)** zum Rohrreaktor **(R)** zur Rückführung der im Dreiphasenseparator **(S)** abgetrennten Gasphase,
IX. sowie eine Rückführungsleitung **(RL3)** von der ersten Trennvorrichtung **(K1)** zum Rohrreaktor **(R)** zur Rückführung der C₃-C4-Kohlenwasserstofffraktion,
wobei die Komponenten I. bis IV. in Reihe geschaltet sind, und
wobei die Anlage eine vierte Trennvorrichtung **(K4)** enthält, die geeignet ist, eine Gasphase, enthaltend C₁- bis C₅- Kohlenwasserstoffe, in eine C₁- bis C₂- und eine C₃- bis C₅- Kohlenwasserstofffraktion zu trennen und
eine Verbindungsleitung **(VL6)** vom Dreiphasenseparator **(S)** zur vierten Trennvorrichtung **(K4),** die geeignet ist, ein Kohlenwasserstoffgas, enthaltend Kohlenwasserstoffe mit 1 bis 5 Kohlenstoffatomen zu transportieren und
eine Verbindungsleitung **(VL4)** von der vierten Trennvorrichtung **(K4)** zur ersten Trennvorrichtung **(K1),** die geeignet ist, C₃- bis C₅- Kohlenwasserstoffe zu transportieren.

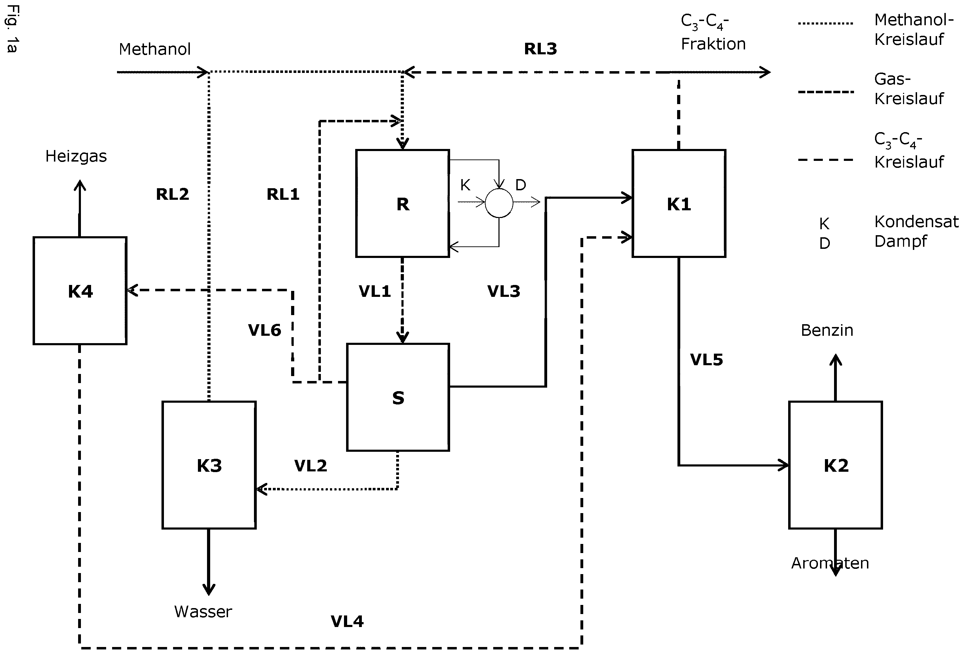

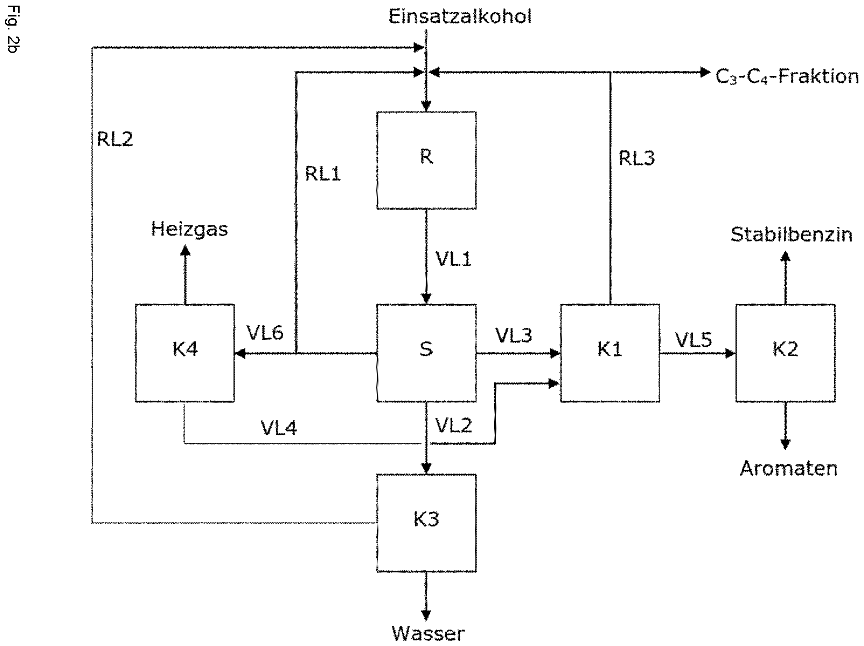

In einer Variante (Fig. 1a, 2b) umfasst die erfindungsgemäße Anlage folgende Komponenten:
a. Isothermen Rohrreaktor **(R)** zur katalytischen Umsetzung von Alkoholen in ein Produktgemisch, enthaltend ein Kohlenwasserstoffgemisch und Wasser,
b. Dreiphasenseparator **(S)** zur Auftrennung des im Rohrreaktor **(R)** erhaltenen Produktgemisches in eine flüssige Kohlenwasserstoffphase, eine wässrige Phase, enthaltend nicht umgesetzten Alkohol, und eine Gasphase,
c. eine erste Trennvorrichtung **(K1),** die geeignet ist, eine flüssige Kohlenwasserstoffphase, enthaltend Kohlenwasserstoffe mit 3 bis 11 Kohlenstoffatomen, in eine C₃-C₄-Fraktion und eine C₅₊-Fraktion aufzutrennen,
d. eine zweite Trennvorrichtung **(K2),** die geeignet ist, eine Kohlenwasserstofffraktion, enthaltend Kohlenwasserstoffe mit 5 bis 11 C-Atomen, in eine durolhaltige schwere Aromaten-Fraktion und eine Stabilbenzinfraktion zu trennen,
e. eine dritte Trennvorrichtung **(K3),** die zur Trennung einer wässrigen Phase, enthaltend Alkohol, in Wasser und Alkohol geeignet ist, wobei die Alkohole bevorzugt 1 bis 3 Kohlenstoffatome enthalten,
f. eine vierte Trennvorrichtung **(K4),** die geeignet ist, eine Gasphase, enthaltend C₁-C₅-Kohlenwasserstoffe, in eine C₁-C₂- und eine C₃-C₅-Kohlenwasserstofffraktion zu trennen,
g. eine erste Verbindungsleitung **(VL1)** vom Reaktor **R** zum Dreiphasenseparator **S** zum Transport des Reaktionsproduktes, enthaltend C₁-C₁₁-Kohlenwasserstoffe, Wasser und Alkohol,
h. eine zweite Verbindungsleitung **(VL2)** vom Dreiphasenseparator **(S)** zur dritten Trennvorrichtung **(K3),** die geeignet ist, eine wässrige Phase, enthaltend Alkohol, zu transportieren,
i. eine dritte Verbindungsleitung **(VL3)** vom Dreiphasenseparator **(S)** zur ersten Trennvorrichtung **(K1),** die geeignet ist, eine flüssige Kohlenwasserstoffphase, enthaltend Kohlenwasserstoffe mit 3 bis 11 Kohlenstoffatomen, zu transportieren,
j. eine vierte Verbindungsleitung **(VL4)** von der vierten Trennvorrichtung **(K4)** zur ersten Trennvorrichtung **(K1),** die geeignet ist, C₃-C₅-Kohlenwasserstoffe zu transportieren,
k. eine fünfte Verbindungsleitung **(VL5)** von der ersten Trennvorrichtung **(K1)** zur zweiten Trennvorrichtung **(K2),** die geeignet ist, eine flüssige Kohlenwasserstoffphase, enthaltend Kohlenwasserstoffe mit 5 bis 11 Kohlenstoffatomen, zu transportieren,
l. eine sechste Verbindungsleitung **(VL6)** vom Dreiphasenseparator **(S)** zur vierten Trennvorrichtung **(K4),** die geeignet ist, ein Kohlenwasserstoffgas, enthaltend Kohlenwasserstoffe mit 1 bis 5 Kohlenstoffatomen, zu transportieren,
m. eine erste Rückführungsleitung **(RL1)** vom Dreiphasenseparator **(S)** zum Reaktor **(R)** zur Rückführung eines Teils der im Dreiphasenseparator **(S)** abgetrennten Gasphase,
n. eine zweite Rückführungsleitung **(RL2)** von der dritten Trennvorrichtung **(K3)** zum Reaktor **(R)** zur Rückführung des nicht umgesetzten Alkohols,
o. eine dritte Rückführungsleitung **(RL3)** von der ersten Trennvorrichtung **(K1)** zum Reaktor **(R)** zur Rückführung der C₃-C₄-Kohlenwasserstofffraktion.

Die Ausführungsform ist beispielhaft in Fig. 2b (1a) dargestellt, welche ein Blockfließbild einer Anlage zur Erzeugung von Benzin aus Einsatzalkohol zeigt, wobei eine Rückführung von Kreislaufgas, der C₃-C₄-Fraktion und des nicht umgesetzten Alkohols erfolgt:
Erfindungsgemäß sind die Komponenten Rohrreaktor **R,** Dreiphasenseparator **S,** erste Trennvorrichtung **K1** und zweite Trennvorrichtung **K2** in Reihe geschaltet.

In einer Ausführungsform sind mindestens die Trennvorrichtungen K1 und K2 Destillationskolonnen. In einer weiteren Ausführungsform sind alle Trennvorrichtungen K1 bis K4 Destillationskolonnen.

Der Rohrreaktor **R** ist über das Reaktionsprodukt in Verbindungsleitung **VL1** mit dem Separator **S** verbunden, der Separator **S** über die flüssige Kohlenwasserstoffphase in Verbindungsleitung **VL3** mit der ersten Trennvorrichtung **K1,** beispielsweise einer Benzinstabilisierungskolonne, und die Trennvorrichtung **K1** ist über die Flüssiggasfraktion C₃-C₄ (Kopfprodukt der Trennvorrichtung **K1)** in einer Rückführungsleitung **RL3** mit dem Reaktor **R** und über deren Sumpfprodukt über eine Verbindungsleitung **VL5** direkt mit der zweiten Trennvorrichtung **K2,** beispielsweise einer Benzintrennkolonne, verbunden.

Erfindungsgemäß enthält die Anlage eine Rückführungsleitung **(RL3)** von der ersten Trennvorrichtung **(K1)** zum Reaktor **(R)** zur Rückführung der C₃-C₄- Kohlenwasserstofffraktion.

In einer Ausführungsform ist der Separator **S** über die wässrige Phase in der Verbindungsleitung **VL2** direkt mit einer dritten Trennvorrichtung **K3,** beispielsweise mit einer Methanolkolonne, verbunden, deren Kopfprodukt (Alkohol) in der Rückführungsleitung **RL2** mit dem Reaktor **R** verbunden ist.

Erfindungsgemäß ist der Dreiphasenseparator **S** über das Ci-Cs-Kohlenwasserstoffgas in der Verbindungsleitung **VL6** mit einer Trennvorrichtung **K4,** beispielsweise mit einer Separationsgaskolonne, verbunden.

Erfindungsgemäß ist der Dreiphasenseparator **S** über die flüssige Kohlenwasserstoffphase aus dem Separator **S** in der Verbindungsleitung **VL3** mit der Trennvorrichtung **K1** verbunden.

Die Trennvorrichtung **K4** ist über die Kohlenwasserstofffraktion C₃ bis C₅ in der Verbindungsleitung **VL4** direkt mit der Trennvorrichtung **K1** verbunden.

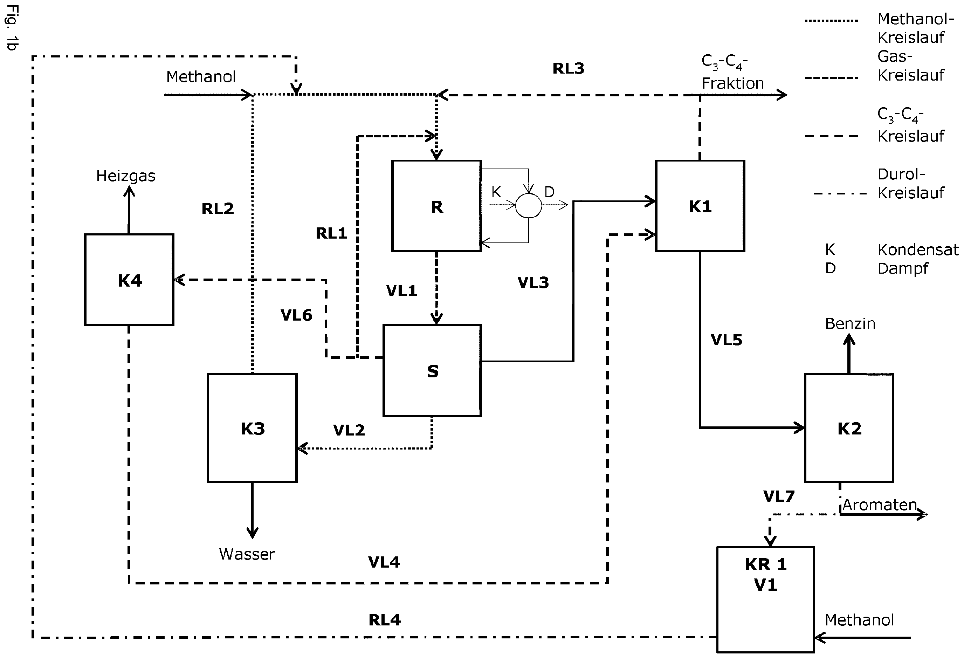

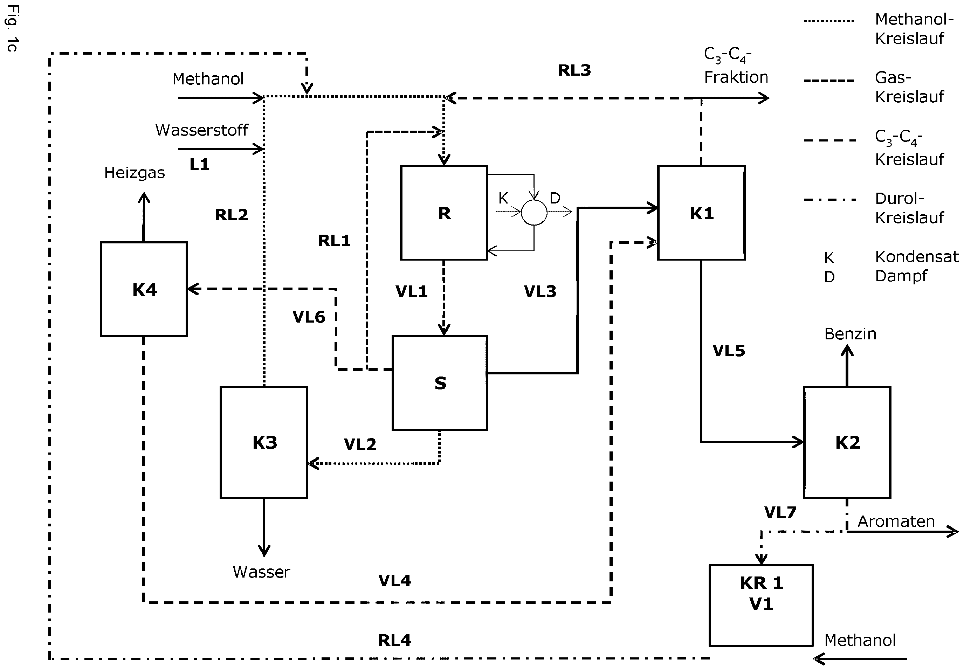

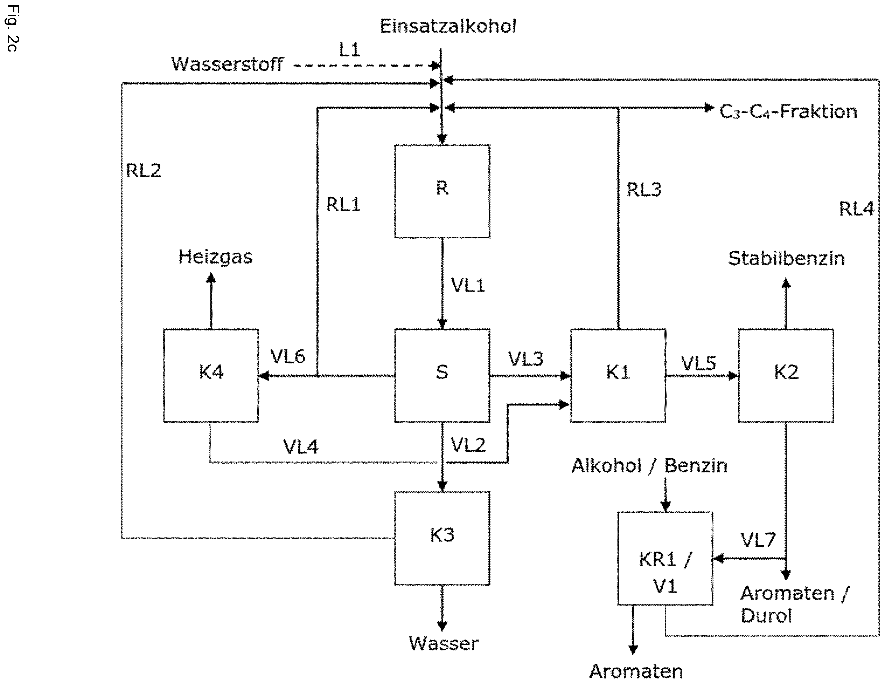

In einer Ausführungsform (Fig. 2c, 1b, 1c) enthält die Anlage zusätzlich einen Kristallisator **KR1,** der dazu geeignet ist, Durol aus einer durolhaltigen Fraktion oder schweren Aromatenfraktion auszukristallisieren.

In einer Ausführungsform ist die Trennvorrichtung **K2** dabei direkt über eine Verbindungsleitung **VL7** mit dem Kristallisator **KR1** verbunden.

In einer Ausführungsform (Fig. 2c, 1b, 1c) enthält die Anlage zusätzlich eine Vorrichtung **V1** zum Auflösen des im Kristallisator **KR1** abgetrennten Durols und eine vierte Rückführungsleitung **RL4** vom Kristallisator **KR1** mit Vorrichtung **V1** zum Reaktor **R,** die geeignet ist, gelöstes Durol zu transportieren. Dabei ist der Kristallisator **KR1** über das in der Vorrichtung **V1** gelöste Durol in einer Rückführungsleitung **RL4** mit dem Reaktor R verbunden.

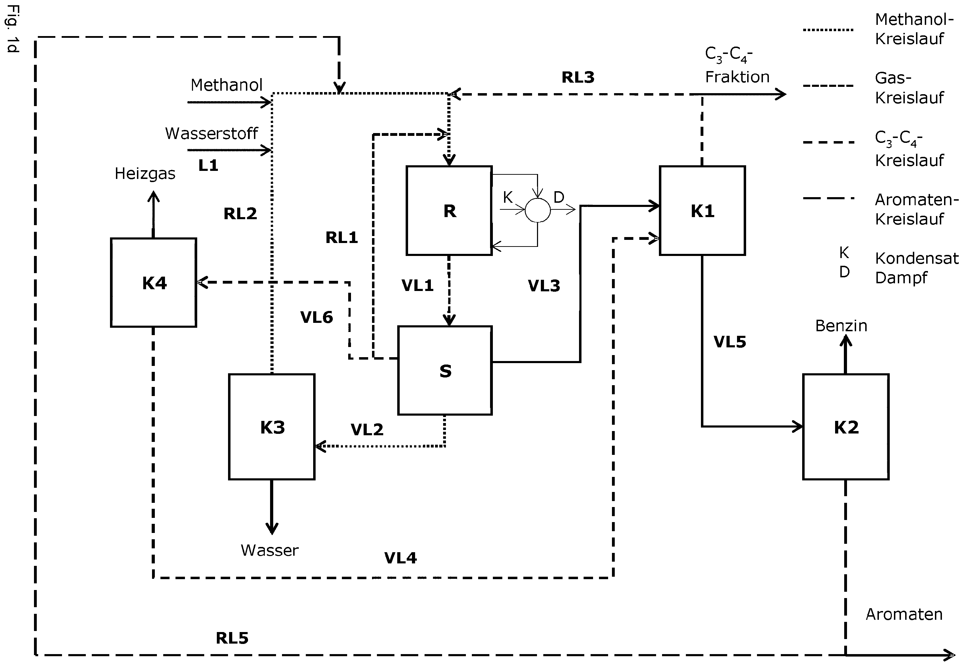

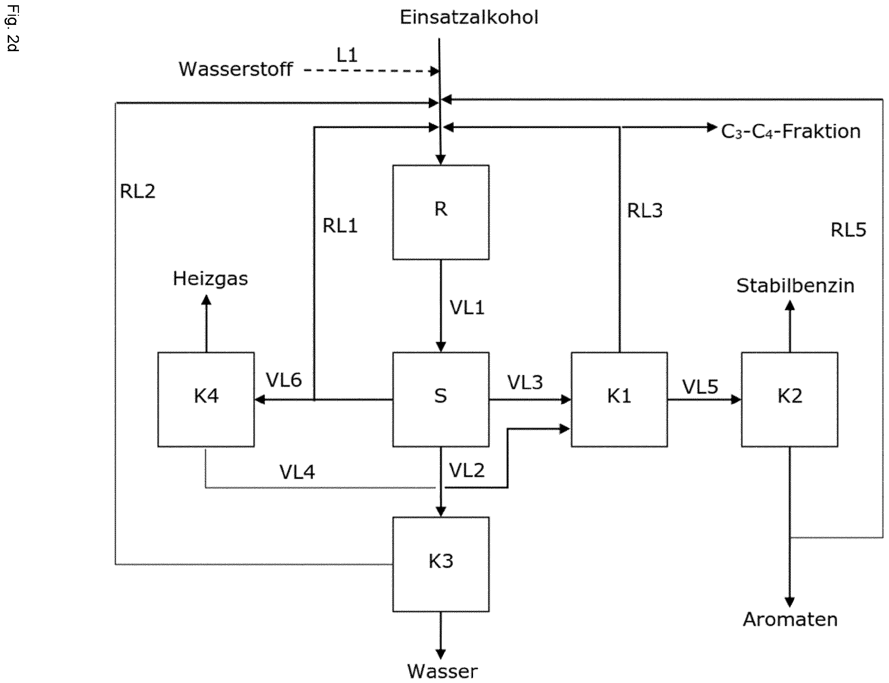

In einer Ausführungsform (Fig. 2c, 2d, 1c, 1d), bei der Erzeugung eines Produktbenzins mit einem hohen iso-Paraffingehalt und einem niedrigen Aromatengehalt, ist die Anlage optional mit einer zusätzlichen externen Wasserstoffquelle und Zuspeisung des Wasserstoffs in einer Leitung **L1** zum Reaktor **R** ausgestattet. Die zusätzliche Zuspeisung von Wasserstoff dient der Absättigung der wasserstoffarmen olefinischen und aromatischen Komponenten mit Wasserstoff und deren Umwandlung zu n- und iso-Paraffinen und Naphthenen und der Verringerung der Koksbildung auf der Katalysatoroberfläche. Eine verminderte Koksbildung erhöht die Zwischenregenerierdauer und die gesamte Lebensdauer des Katalysators.

In einer Ausführungsform (Fig. 2d, 1d) zur Verringerung der Aromatenbildung kann, insbesondere zu Beginn eines Reaktionszyklusses, auch ein Teil der methylierten Aromatenfraktion der Trennvorrichtung **K2,** beispielsweise einer Benzintrennkolonne, ohne vorherige Abtrennung des Durols, zum Reaktor zurückgeführt werden. Dabei ist die zweite Trennvorrichtung **K2** über dessen Sumpfprodukt, der schweren methylierten Aromatenfraktion (bzw. eines Teils davon), in einer Rückführungsleitung **RL5** mit dem Reaktor **R** verbunden. Durch diese Methode wird der Aufbau eines Carbonpools auf der aktiven Katalysatoroberfläche gefördert, wodurch seine Aktivität abgeschwächt wird und weniger Aromaten C₆ bis Cg gebildet werden. Es wird die Alterung des Katalysators verringert und seine Lebensdauer erhöht.

Für die Realisierung der Erfindung ist es auch zweckmäßig, die vorbeschriebenen Ausführungsformen und Merkmale der Ansprüche zu kombinieren.

### Ausführungsbeispiele

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.
- Fig. 1a:: Anlage zur Erzeugung von Benzin variabler Zusammensetzung
- Fig. 1b:: Anlage mit Durolrückführung zur Erzeugung von Benzin mit hohem iso-Paraffingehalt ohne Zuspeisung von Wasserstoff
- Fig. 1c:: Anlage mit Durolrückführung zur Erzeugung von Benzin mit hohem iso-Paraffingehalt mit Zuspeisung von Wasserstoff
- Fig. 1d:: Anlage mit Aromatenrückführung zur Erzeugung von Benzin mit hohem iso-Paraffingehalt und mit Zuspeisung von Wasserstoff

Weiterhin zeigen die Figuren 2b bis 2d vereinfachte Blockfließbilder der erfindungsgemäßen Anlage.

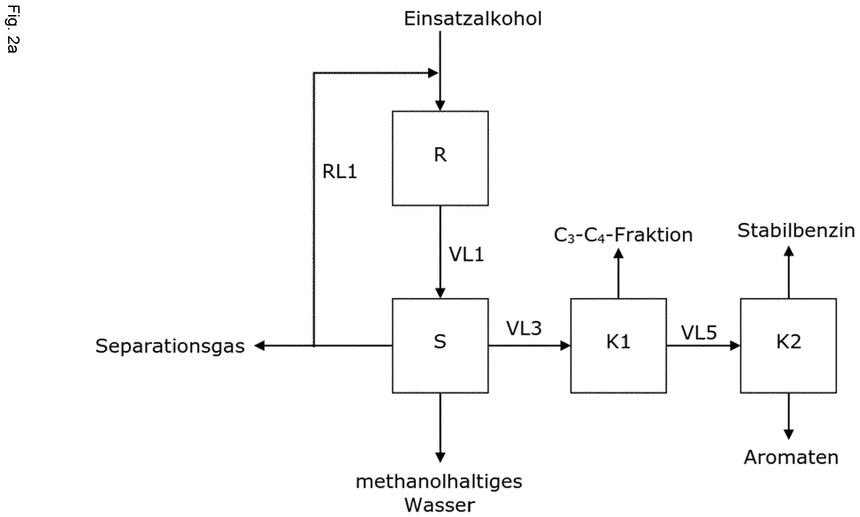

Dabei zeigen die Figuren 2b, 2c und 2d Verfahrens der Erzeugung von Benzin mit hohem iso-Paraffingehalt. Die Figur 2a zeigt ein vergleichsweises Verfahren zur Erzeugung von Benzin aus Alkohol, beispielhaft aus Methanol.
- Fig. 2a: - Vereinfachtes Blockfließbild einer Vergleichsanlage zur Erzeugung von Benzin aus Einsatzalkohol mit Rückführung von Kreislaufgas
- Fig. 2b: - Blockfließbild der erfindungsgemäßen Anlage zur Erzeugung von Benzin aus Einsatzalkohol mit Rückführung von Kreislaufgas, der C₃-C₄-Fraktion und des nicht umgesetzten Alkohols
- Fig. 2c: - Blockfließbild der erfindungsgemäßen Anlage zur Erzeugung von Benzin aus Einsatzalkohol mit Rückführung von Kreislaufgas, der C₃-C₄- Fraktion, des nicht umgesetzten Alkohols und von gelöstem Durol, optional - Zuspeisung von Wasserstoff
- Fig. 2d: - Blockfließbild der erfindungsgemäßen Anlage zur Erzeugung von Benzin aus Einsatzalkohol mit Rückführung von Kreislaufgas, der C₃-C₄-Fraktion, des nicht umgesetzten Alkohols und von Aromaten, optional - Zuspeisung von Wasserstoff

### Ausführungsbeispiel 1:

**Fig. 1c****,** **1b** **/** **Fig. 2c** zeigt ein Blockfließbild des erfindungsgemäßen Verfahrens der Erzeugung von hochoktanigem Benzin mit hohem iso-Paraffingehalt aus Einsatzalkohol (in diesem Ausführungsbeispiel = Methanol) mit Durolrückführung.

Die Trennvorrichtungen **K1** bis **K4** sind in diesem Fall Kolonnen.

Zur Herstellung eines hochoktanigen Benzins wird Methanol-Feed (100% MeOH) mit dem rückgeführten **(RL2),** im Synthesereaktor **R,** nicht umgesetzten Methanol (aus der Trennvorrichtung **K3),** vermischt und in den Gaskreislauf in flüssiger Form eingespeist. Gleichzeitig werden auch die olefinhaltige Flüssiggasfraktion einer Zusammensetzung von 2-4 Ma.-% Ethylen, 15-25 Ma.-% Propylen, 30-50 Ma.-% Butylen, 5-15 Ma.-% Butan, 2-4 Ma.-% Penten, 2-6 Ma.-% i-Pentan, 5-15 Ma.-% DME und 2-7 Ma.-% Methanol aus der Trennvorrichtung **K1** in **RL3** und das im Methanol gelöste Durol (ca. 3 - 5 Ma.-%, bezogen auf die in den Reaktor eintretende Methanolmenge) aus dem Kristallisator **KR1** und der Vorrichtung **V1** in **RL4** in den Gaskreislauf eingespeist (nur zu Beginn jeder Reaktionsphase).

Die genannten Flüssigkeiten Methanol, C₃-C₄-Fraktion und das im Methanol gelöste Durol werden im Gegenstrom zum heißen Reaktionsprodukt in einem Wärmetauscher verdampft. Danach wird der Gesamtstrom - bestehend aus Kreislaufgas und den gasförmigen Strömen des Methanols, der C₃-C₄-Fraktion und des Durols - in einem Ofen bis zur geforderten Reaktoreintrittstemperatur von 340 °C aufgeheizt. Der Ofen wird dabei mit Heizgas, welches Bestandteil des Reaktionsproduktes ist und zuvor in einer Destillationskolonne **K4** als Kopfprodukt abgetrennt wurde, beheizt.

Das gasförmige Gemisch tritt in den Rohrreaktor **R** ein. Mit Hilfe eines Kompressors, welcher das Kreislaufgas zum Reaktor fördert, kann der Mengenstrom des Kreislaufgases so eingestellt werden, dass sich ein Stoffmengenanteil des Methanols im Einsatzgas in den Reaktor von 40 bis 45 Mol.-% ergibt.

Im Benzinsynthesereaktor **R** findet eine exotherme Reaktion der Umwandlung des Methanols und bestimmter Komponenten im Einsatzgas-wie DME und Olefine - in Kohlenwasserstoffe, die hauptsächlich im Siedebereich von Benzin liegen, statt. Das Methanol setzt sich bei den eingestellten Bedingungen zu 95 % um. Die dabei freigesetzte Wärme wird im Reaktor durch einen internen Wärmetauscher, welcher einen geeigneten Wärmeträger enthält, abgeführt. Der Wärmeträger wird in einem separaten Wärmetauscher abgekühlt. Dabei wird Dampf eines Druckes von 30 bar aus Kondensat erzeugt und in den Trennvorrichtungen **K1, K2** und **K3,** die der Produkttrennung dienen, als Heizmedium der Sumpferhitzer genutzt. Das Kondensat aus den Sumpferhitzern der Trennvorrichtungen strömt zum Kühler des Wärmeträgers des Reaktors zurück.

Das Gemisch an Einsatzstoffen wird mit einem Druck von 7 barg in den Reaktor eingespeist. Die Umsetzung des Methanols erfolgt in einem isothermen Rohrreaktor **R** mit einem Katalysatorbett aus einem ZSM-5 Katalysator, welcher im Eingangsbereich der Einsatzstoffe mit 10-25 % Inertmaterial verdünnt wurde. Die anderen Katalysatorbereiche sind in unterschiedlichen Verdünnungsgraden mit Inertmaterial verdünnt.

Nachfolgend werden die erfindungsgemäßen Verfahrensschritte anhand von Ausführungsbeispielen erläutert.
a. Das 340 °C heiße Reaktionsprodukt aus dem Benzinsynthesereaktor **R** wird im Gegenstrom zum 50 °C kalten Einsatzprodukt in Wärmetauschern bis auf 75 °C gekühlt, anschließend wird das Reaktionsprodukt weiter in einem Kühler, welcher ein Wasserkühler und ein Kaltwasserkühler oder eine Kombination aus Luftkühler und Wasser- und Kaltwasserkühler sein kann, bis auf 5 °C gekühlt und in der Verbindungsleitung **VL1** dem Dreiphasenseparator **S** zugeführt, wobei das Wasser, das nicht umgesetzte Methanol und die kondensierbaren Kohlenwasserstoffe auskondensieren.
b. Der Separator **S** trennt das abgekühlte Reaktionsprodukt in 3 Phasen - eine wässrige Phase, eine flüssige Kohlenwasserstoffphase und eine gasförmige Kohlenwasserstoffphase.
c. Die wässrige Phase aus dem Separator **S** wird in der Verbindungsleitung **VL2** zur dritten Kolonne **K3** geführt und in Methanol und Wasser aufgetrennt. Kopfprodukt der Kolonne **K3** ist das im Reaktor **R** nicht umgesetzte Methanol mit einem Restgehalt an Wasser, welches zum Teil als Rücklauf zum Kolonnenkopf rückgeführt wird. Die Kolonne **K3** trennt das Methanol-Wasser bei einem Kopfdruck von 1,2 barg, einer Kopftemperatur von 85 °C und einer Sumpftemperatur von 130 °C.
d. Die im Reaktor **R** nicht umgesetzte Methanolmenge wird der Kolonne **K3** als Kopfprodukt (Wassergehalt: ca. 7 Ma.-%) entnommen, strömt dann in der Rückführungsleitung **RL2** zum Gaskreislauf und wird mit dem Einsatzalkohol (100 % Methanol) vermischt, bevor beides in den Gaskreislauf eingespeist wird.
   Das Sumpfprodukt der Trennvorrichtung **K3** ist das vom Methanol gereinigte Wasser. Der Reinigungsgrad hängt von der Trennleistung der Trennvorrichtung **K3** ab. Der Methanolgehalt im gereinigten Wasser beträgt < 5 ppm.
e. Ein Teil der Gasphase wird mit Hilfe eines Kompressors im Kreislauf in der Rückführungsleitung **RL1** wieder zurück zum Reaktor **R** geführt (erster Teil der Gasphase). Der Kompressor fördert mit einem Austrittsdruck im Bereich von 9 bis 11 barg das Gas zum Reaktor **R** und gleicht die Druckverluste im Gaskreislauf aus.
f. Ein Teilstrom der Gasphase aus dem Separator (zweiter Teil der Gasphase) wird aus dem Gaskreislauf ausgeschleust und mit Hilfe eines weiteren Kompressors in der Verbindungsleitung **VL6** zur vierten Trennvorrichtung, einer Separationsgaskolonne **K4,** gefördert.
   Der Druck in der Kolonne **K4** liegt bevorzugt bei ca. 15 bar. Die Separationsgaskolonne **K4** hat keinen Sumpferhitzer. Das aus dem Kopf der Kolonne austretende Kohlenwasserstoffgas (hauptsächlich C₁-C₂-Kwst. sowie geringe Mengen an C₃-C₄-Kwst.) wird gekühlt, bevorzugt bis auf-20 °C. Zur Kühlung im Kopfproduktkühler wird ein herkömmliches Kühlmedium aus einer Kältemaschine verwendet.
   Das aus dem Kopfproduktseparator austretende gasförmige Produkt (C₁-C₂-Kohlenwasserstoffe) wird als Heizgas im Ofen der Vorheizung des Einsatzstoffes des Rohrreaktors **R** verwendet.
   Das Flüssigprodukt aus dem Kopfproduktseparator der Trennvorrichtung **K4** strömt teilweise als Rücklauf zur Trennvorrichtung **K4** zurück, der andere Teil wird als Flüssigprodukt entnommen und mit dem erhaltenen Sumpfprodukt der Trennvorrichtung **K4** vermischt.
g. Das Gemisch besteht aus C₃-C₅-Kohlenwasserstoffen und wird in der Verbindungsleitung **VL4** zur Trennvorrichtung **K1,** einer Stabilisierungskolonne, gegeben. Haupteinsatzstoff der Stabilisierungskolonne **K1** ist die flüssige Kohlenwasserstoffphase des Reaktionsproduktes, die in Verbindungsleitung **VL3** vom Separator **S** zur Kolonne **K1** strömt. Der Druck im Separator **S** entspricht dem Reaktoreintrittsdruck abzüglich der Druckverluste im Reaktor und in den Wärmetauschern zur Kühlung des Reaktionsproduktes. Er liegt bei ca. 2,2 barg.
h. Die Flüssigphase aus dem Separator **S** wird auf 120 °C aufgewärmt und in die Stabilisierungskolonne **K1** eingespeist.
   Die Kolonne **K1** dient der Stabilisierung der flüssigen Kohlenwasserstoffe, das bedeutet, dass die leicht siedenden Komponenten C₃-C₄ und teilweise C₅ im Kopf der Kolonne abgetrennt werden. Ziel der Trennung in Kolonne **K1** ist die Einstellung des Dampfdruckes des Benzins entsprechend der Spezifikation.
   Die Kolonne **K1** trennt bei einem Druck von 15-16 barg die flüssigen Kohlenwasserstoffe in eine Flüssiggasfraktion (hauptsächlich C₃-C₄ und DME) und eine C₅₊-Fraktion. Die Temperatur des Sumpferhitzers beträgt ca. 220 °C. Die C₅₊-Kohlenwasserstoffe aus dem Kolonnensumpf werden über die Verbindungsleitung **VL5** in die Trennvorrichtung **K2,** eine Benzintrennkolonne, eingespeist.
i. Der Kopfproduktkühler der Stabilisierungskolonne **K1** kühlt das Kopfprodukt (Flüssiggasfraktion - hauptsächlich C₃-C₄ und DME) auf ca. 20 °C. Das erhaltene flüssige Kopfprodukt wird mit Hilfe der Rückführungsleitung **RL3** in den Gaskreislauf eingespeist, ein Teil wird als Produkt entnommen.
j. Die Benzintrennkolonne **K2** hat einen Kopfdruck von 0,2 barg und eine Kopftemperatur von 70 °C. Die Temperatur des Sumpferhitzers beträgt ca. 240 °C. In Trennvorrichtung **K2** wird Stabilbenzin als Kopfprodukt erhalten und als Sumpfprodukt eine schwere Kohlenwasserstofffraktion mit einem hohen Gehalt an Durol (ca. 90 Ma.-%) sowie anderen Alkylaromaten mit Kohlenstoffzahlen von 10 und 11. Durol (1,2,4,5-Tetramethylbenzol) ist ein Alkylaromat mit einer niedrigen Erstarrungstemperatur von ca. 79 °C und kann durch Kristallisation von den anderen aromatischen Verbindungen separiert werden. Dazu wird das Sumpfprodukt der Trennvorrichtung **K2** in der Verbindungsleitung **VL7** einem Kristallisator **KR1** zugeführt.
   In dem Kristallisator **KR1** wird durch Abkühlung des Sumpfproduktes auf eine Temperatur von < 79 °C, bevorzugt 60 °C, das Durol von den übrigen Komponenten abgetrennt. Das feste Durol wird in der Kristallisationseinheit **KR1** separiert und ein Teil des Durols dann in der Vorrichtung **V1** in Methanol gelöst. Als flüssige Lösung wird dann das Methanol-Durol-Gemisch in der Rückführungsleitung **RL4** in den Gaskreislauf eingespeist.

Das Produktbenzin hat folgende Zusammensetzung:

**Tabelle 1: Zusammensetzung des Benzins**

| | Ma.-% | Vol.-% |
|---|---|---|
| n-Paraffine | 1,5 | 1,8 |
| iso-Paraffine | 61,6 | 64,2 |
| Olefine | 7,8 | 9,0 |
| Naphthene | 3,3 | 3,2 |
| Aromaten | 25,2 | 21,3 |
| Oxygenate | 0,6 | 0,5 |
| | 100,0 | 100,0 |

Der Dampfdruck des Benzins beträgt 45 kPa.

### Vergleichsbeispiel 2 - Erzeugung eines aromatenreichen Benzins:

**Fig. 2a** zeigt ein Blockfließbild des erfindungsgemäßen Verfahrens der Erzeugung von Benzin mit Rückführung von Kreislaufgas. Bei Einstellung geeigneter Parameter kann mit diesem Verfahren ein aromatenreiches hochoktaniges Benzin aus Methanol hergestellt werden, wie das folgende Beispiel zeigt.

Die Vergleichsanlage beinhaltet folgende Hauptausrüstungen: einen Reaktor **R** mit Kühlung der Reaktionszone zur Synthese von Kohlenwasserstoffen aus Methanol, einen Separator **S** zur Trennung des abgekühlten Reaktionsproduktes in flüssige Kohlenwasserstoffe, Wasser mit Methanol und eine Gasphase, eine Trennvorrichtung **K1** zur Abtrennung der Flüssiggaskomponenten aus den flüssigen Kohlenwasserstoffen und eine Trennvorrichtung **K2** zur Trennung der flüssigen Kohlenwasserstoffe aus dem Sumpf der Trennvorrichtung **K1** in eine Stabilbenzinfraktion und eine Fraktion schwerer methylierter Aromaten.

In der Anlage wird ein Teil der Gasphase (Hauptteil) aus dem Separator **S** in der Rückführungsleitung **RL1** zum Reaktor rückgeführt.

Weiterhin enthält die Anlage einen Verdichter zur Aufrechterhaltung des Gaskreislaufes zum / vom Reaktor sowie Behälter und Wärmetauscher u. a..

Im Vergleichsbeispiel 2 wird Einsatzalkohol in Form von 100 Ma.-% Methanol eingespeist. Im Reaktor der Benzinsynthese wird ein zeolithhaltiger Katalysator des Typs ZSM-5 eingesetzt.

Die Bedingungen am Eintritt in den Reaktor sind 375 °C und 6 barg. Die LHSV des Methanols beträgt 1,0 h⁻¹. Der Stoffmengenanteil des Methanols im Einsatzgas in den Reaktor beträgt 55,1 Mol.-%. Der Umsatzgrad des Methanols im Reaktor beträgt 100,0 %.

In Tabelle 2 wird die Materialbilanz der Vergleichs-Gesamtanlage gegeben.

**Tabelle 2: Materialbilanz der Vergleichsanlage**

| Eingang | | Ausgang | | |
|---|---|---|---|---|
| | Ma.-% | | Ma.-% | Ma.-% |
| Methanol | 100,0 | Heizgas | 7,5 | 17,2 |
| | | Flüssiggas | 3,5 | 8,0 |
| | | Benzin | 29,6 | 67,6 |
| | | Schwerbenzin | 3,2 | 7,2 |
| | | gesamt | 43,8 | 100,0 |
| | | Wasser | 56,2 | |
| gesamt | 100,0 | gesamt | 100,0 | |

Die Benzinselektivität (bezogen auf die Masse der erzeugten Kohlenwasserstoffe) beträgt 67,6%.

In Tabelle 3 wird die Zusammensetzung des erzeugten Benzins gegeben.

**Tabelle 3: Zusammensetzung des Benzins**

| | Ma.-% | Vol.-% |
|---|---|---|
| n-Paraffine | 5,5 | 6,4 |
| iso-Paraffine | 42,5 | 48,0 |
| Olefine | 2,4 | 2,7 |
| Naphthene | 8,4 | 8,1 |
| Aromaten | 41,2 | 34,8 |
| Oxygenate | 0 | 0 |
| | 100,0 | 100,0 |

Die Oktanzahl des Benzins beträgt ROZ = 100.

### Bezugszeichen bzw. Abkürzungsliste

- **R**: Isothermer Rohrreaktor zur katalytischen Umsetzung von Alkoholen
- **S**: Dreiphasenseparator
- **K1**: erste Trennvorrichtung, bspw. Benzinstabilisierungskolonne
- **K2**: zweite Trennvorrichtung, bspw. Benzintrennkolonne
- **K3**: dritte Trennvorrichtung, bspw. Methanolkolonne
- **K4**: Separationsgastrennvorrichtung, Separationsgaskolonne
- **KR1**: Kristallisator
- **V1**: Vorrichtung zum Auflösen von Durol
- **VL1**: Verbindungsleitung vom Reaktor **R** zum Dreiphasenseparator **S** zum Transport des Reaktionsproduktes, enthaltend C₁-C₁₁-Kohlenwasserstoffe, Wasser und Alkohol
- **VL2**: Verbindungsleitung vom Dreiphasenseparator **S** zur Trennvorrichtung **K3** zum Transport der wässrigen Phase, enthaltend Alkohol
- **VL3**: Verbindungsleitung vom Dreiphasenseparator **S** zur Trennvorrichtung **K1** zum Transport der flüssigen C₃-C₁₁-Kohlenwasserstoffe
- **VL4**: Verbindungsleitung von der Trennvorrichtung **K4** zur Trennvorrichtung **K1** zum Transport der flüssigen C₃-C₅-Kohlenwasserstoffe
- **VL5**: Verbindungsleitung von der Trennvorrichtung **K1** zur Trennvorrichtung **K2** zum Transport der flüssigen C₅-C₁₁-Kohlenwasserstoffe
- **VL6**: Verbindungsleitung vom Dreiphasenseparator **S** zur Trennvorrichtung **K4** zum Transport der Kohlenwasserstoffgase
- **VL7**: Verbindungsleitung von der Trennvorrichtung **K2** zum Kristallisator **KR1** zum Transport der schweren Aromatenfraktion
- **RL1**: Rückführungsleitung vom Dreiphasenseparator **S** zum Reaktor **R** zur Rückführung des Kohlenwasserstoffgases
- **RL2**: Rückführungsleitung von der Trennvorrichtung **K3** zum Reaktor **R** zur Rückführung des nicht umgesetzten Alkohols
- **RL3**: Rückführungsleitung von der Trennvorrichtung **K1** zum Reaktor **R** zur Rückführung der C₃-C₄-Kohlenwasserstoffe
- **RL4**: Rückführungsleitung von der Vorrichtung zum Auflösen von Durol **V1** zum Reaktor **R** zur Rückführung des Durols
- **RL5**: Rückführungsleitung von der Trennvorrichtung **K2** zum Reaktor **R** zur Rückführung der schweren Aromatenfraktion

## Patentansprüche

1. Verfahren zur Herstellung eines synthetischen Benzins mit hohem iso-Paraffingehalt im Bereich 50-65 Ma% und mit niedrigem Aromatengehalt im Bereich 20-35 Ma% mit den Schritten:
I. katalytische Umsetzung von Einsatzalkohol in einem Einsatzgas in ein Produktgemisch, enthaltend Wasser und ein Kohlenwasserstoffgemisch aus Olefinen, n-Paraffinen, iso-Paraffinen, Aromaten und Naphthenen, in einem isothermen Rohrreaktor, enthaltend einen Katalysator,
wobei die Reaktionstemperatur im Rohrreaktor zwischen 300 und 370 °C liegt,
wobei der Einsatzalkohol einen Wasseranteil von weniger als 20 Ma.-% aufweist,
II. Auftrennung des in Schritt **I)** erhaltenen Produktgemisches in
- eine flüssige Kohlenwasserstoffphase,
- eine wässrige Phase, enthaltend den nicht umgesetzten Alkohol,
und
- eine Gasphase, enthaltend C₁- bis C₅- Kohlenwasserstoffe,
III. Rückführung der in Schritt **II**) erhaltenen Gasphase zu Schritt **I),**
IV. Auftrennung der in Schritt **II**) erhaltenen flüssigen Kohlenwasserstoffphase in
- eine olefinhaltige C₃- und C₄-Kohlenwasserstofffraktion und
- eine Benzinkohlenwasserstofffraktion (= C₅₊-Fraktion), enthaltend eine durolhaltige schwere Aromatenfraktion,
V. Auftrennung der in Schritt **IV)** erhaltenen Benzinkohlenwasserstofffraktion in eine durolhaltige schwere Aromatenfraktion und eine Stabilbenzinfraktion,
wobei in Schritt **I)** die Alkoholbelastung des Katalysators 2 bis 5 m³_{Alkohol}/hm³_{Kat} beträgt,
wobei zur Erzeugung eines iso-Paraffin-reichen Benzins der Katalysator im Eintrittsbereich des Alkohols partiell mit 5-30 Massen-% Inertmaterial, bezogen auf die Gesamtmasse an Katalysator und Inertmaterial, verdünnt ist, und
wobei die in Schritt **IV)** abgetrennte olefinhaltige C₃- bis C₄-Kohlenwasserstofffraktion als Einsatzstoff zu Schritt **I)** zurückgeführt wird,
wobei in Schritt **I)** der Stoffmengenanteil des umzusetzenden Alkohols am Rohrreaktoreintritt 25 bis 50 Mol-% umfasst, gemessen an der Gesamtstoffmenge des Einsatzgases, umfassend den Einsatzalkohol und rückgeführte Komponenten.

2. Verfahren nach Anspruch 1, **dadurch gekennzeichnet, dass** die in Schritt **II**) erhaltene wässrige Phase in Wasser und Alkohol getrennt wird und der Alkohol zu Schritt **I)** zurückgeführt wird.

3. Verfahren nach einem der Ansprüche 1 bis 2, **dadurch gekennzeichnet, dass** von einem Teil der in Schritt **II**) erhaltenen Gasphase die C₃- bis C₅- Kohlenwasserstofffraktion abgetrennt wird und diese mit der in Schritt **II**) erhaltenen flüssigen Kohlenwasserstoffphase vereint wird.

4. Verfahren nach einem der Ansprüche 1 bis 3, **dadurch gekennzeichnet, dass** zur Erzeugung eines iso-Paraffin-reichen Benzins ein Teil der in Schritt **V)** erhaltenen schweren durolhaltigen Aromatenfraktion zu Schritt **I)** zurückgeführt wird oder dass das Durol aus der in Schritt **V)** gewonnenen durolhaltigen schweren Aromatenfraktion auskristallisiert wird und ein Teil des auskristallisierten Durols in Alkohol und/oder Benzinkohlenwasserstoffen gelöst wird und zu Schritt **I)** zurückgeführt wird.

5. Verfahren nach einem der Ansprüche 1 bis 4, **dadurch gekennzeichnet, dass** zur Erzeugung eines iso-Paraffin-reichen Benzins zusätzlich Wasserstoff in den Rohrreaktor in Schritt **I)** eingespeist wird.

6. Anlage zur Synthese von synthetischem Benzin mit hohem iso-Paraffingehalt im Bereich 50-65 Ma% und mit niedrigem Aromatengehalt im Bereich 20-35 Ma%, umfassend die Komponenten:
I. Isothermer Rohrreaktor **(R),** enthaltend einen Katalysator, zur katalytischen Umsetzung von Alkoholen in ein Produktgemisch, enthaltend ein Kohlenwasserstoffgemisch und Wasser,
wobei der Katalysator im Eintrittsbereich des Alkohols partiell mit 5-30 Massen-% Inertmaterial, bezogen auf die Gesamtmasse an Katalysator und Inertmaterial, verdünnt ist,
II. Dreiphasenseparator **(S)** zur Auftrennung des im Rohrreaktor **(R)** erhaltenen Produktgemischs in eine flüssige Kohlenwasserstoffphase, eine wässrige Phase, enthaltend nicht umgesetzten Alkohol, und eine Gasphase,
III. eine erste Trennvorrichtung **(K1),** die geeignet ist, eine flüssige Kohlenwasserstoffphase, enthaltend Kohlenwasserstoffe mit 3 bis 11 Kohlenstoffatomen, in eine C₃-C₄- Fraktion und eine C₅₊- Fraktion aufzutrennen,
IV. eine zweite Trennvorrichtung **(K2),** die geeignet ist, eine Kohlenwasserstoffphase, enthaltend Kohlenwasserstoffe mit 5 bis 11 C-Atomen, in eine durolhaltige schwere Aromatenfraktion und eine Stabilbenzinfraktion zu trennen,
V. eine Verbindungsleitung **(VL1)** vom Rohrreaktor **R** zum Dreiphasenseparator **S** zum Transport des Reaktionsproduktes, enthaltend C₁-C₁₁-Kohlenwasserstoffe, Wasser und Alkohol,
VI. eine Verbindungsleitung **(VL3)** vom Dreiphasenseparator **(S)** zur ersten Trennvorrichtung **(K1),** die geeignet ist, eine flüssige Kohlenwasserstoffphase, enthaltend Kohlenwasserstoffe mit 3 bis 11 Kohlenstoffatomen, zu transportieren,
VII. eine Verbindungsleitung **(VL5)** von der ersten Trennvorrichtung **(K1)** zur zweiten Trennvorrichtung **(K2),** die geeignet ist, eine C₅₊-Kohlenwasserstofffraktion zu transportieren,
VIII. eine Rückführungsleitung **(RL1)** vom Dreiphasenseparator **(S)** zum Rohrreaktor **(R)** zur Rückführung der im Dreiphasenseparator **(S)** abgetrennten Gasphase,
IX. sowie eine Rückführungsleitung **(RL3)** von der ersten Trennvorrichtung **(K1)** zum Rohrreaktor **(R)** zur Rückführung der C₃-C4-Kohlenwasserstofffraktion,
wobei die Komponenten I. bis IV. in Reihe geschaltet sind, und
wobei die Anlage eine vierte Trennvorrichtung **(K4)** enthält, die geeignet ist, eine Gasphase, enthaltend C₁- bis C₅- Kohlenwasserstoffe, in eine C₁- bis C₂- und eine C₃- bis C₅- Kohlenwasserstofffraktion zu trennen und
eine Verbindungsleitung **(VL6)** vom Dreiphasenseparator **(S)** zur vierten Trennvorrichtung **(K4),** die geeignet ist, ein Kohlenwasserstoffgas, enthaltend Kohlenwasserstoffe mit 1 bis 5 Kohlenstoffatomen zu transportieren und
eine Verbindungsleitung **(VL4)** von der vierten Trennvorrichtung **(K4)** zur ersten Trennvorrichtung **(K1),** die geeignet ist, C₃- bis C₅- Kohlenwasserstoffe zu transportieren.

7. Anlage nach Anspruch 6, **dadurch gekennzeichnet, dass** die Anlage zusätzlich einen Kristallisator **(KR1)** zum Auskristallisieren von Durol aus der durolhaltigen schweren Aromatenfraktion, eine Verbindungsleitung **(VL7)** von der Trennvorrichtung **(K2)** zum Kristallisator **(KR1)** zum Transport einer schweren Aromatenfraktion, eine Vorrichtung **(V1)** zum Auflösen eines Teils des im Kristallisator abgetrennten Durols und eine Rückführungsleitung **(RL4)** von der Vorrichtung **(V1)** zum Rohrreaktor **(R)** zur Rückführung des gelösten Durols enthält.

8. Anlage nach Anspruch 6 oder 7 **dadurch gekennzeichnet, dass** die Anlage zusätzlich eine Rückführungsleitung **(RL5)** von der zweiten Trennvorrichtung **(K2)** zum Rohrreaktor **(R)** zur Rückführung eines Teils der durolhaltigen Aromatenfraktion enthält.

9. Anlage nach einem der Ansprüche 6 bis 8, **dadurch gekennzeichnet, dass** die Anlage eine dritte Trennvorrichtung **(K3)** enthält, die geeignet ist, eine wässrige Phase, enthaltend Alkohol, in Wasser und Alkohol zu trennen und dass eine Verbindungsleitung **(VL2)** vom Dreiphasenseparator **(S)** zur dritten Trennvorrichtung **(K3)** angeordnet ist, die geeignet ist, eine wässrige Phase, enthaltend Alkohol, zu transportieren und dass eine Rückführungsleitung **(RL2)** von der dritten Trennvorrichtung **(K3)** zum Rohrreaktor **(R)** zur Rückführung des nicht umgesetzten Alkohols angeordnet ist.

## Claims

1. A method for the production of a synthetic gasoline with a high isoparaffin content in the range of 50-65 mass% and a low aromatics content in the range of 20-35 mass%, comprising the following steps:
I. catalytic conversion of feed alcohol in a feed gas into a product mixture containing water and a hydrocarbon mixture of olefins, n-paraffins, isoparaffins, aromatics, and naphthenes within an isothermal tubular reactor containing a catalyst,
wherein the reaction temperature within the tubular reactor is between 300 and 370 °C,
wherein the feed alcohol has a water content of less than 20 mass%,
II. separation of the product mixture obtained in step **I)** into:
- a liquid hydrocarbon phase,
- an aqueous phase containing the unconverted alcohol, and
- a gas phase containing C₁- to C₅- hydrocarbons,
III. recycling of the gas phase obtained in step **II**) to step **I),**
IV. separation of the liquid hydrocarbon phase obtained in step **II)** into
- an olefin-containing C₃- to C₄- hydrocarbon fraction and
- a gasoline hydrocarbon fraction (= C₅₊ fraction) containing a durene-containing heavy aromatics fraction,
V. separation of the gasoline hydrocarbon fraction obtained in step **IV)** into a durene-containing heavy aromatics fraction and a stable gasoline fraction,
wherein in step **I)** the alcohol load of the catalyst is 2 to 5 m³_{alcohol}/hm³cₐₜ,
wherein for producing an isoparaffin-rich gasoline, the catalyst is partially diluted in the inlet region of the alcohol with inert material with a mass fraction of 5 to 30 %, based on the total mass of catalyst and inert material, and
wherein the olefin-containing C₃- to C₄- hydrocarbon fraction separated off in step **IV)** is recycled to step **I)** as feed material,
wherein in step **I)** the mole fraction of alcohol, which is to be converted, in the total amount of feed gas at the reactor inlet comprises 25 to 50 mol%, wherein the feed gas comprises the feed alcohol and the recycled components.

2. The method according to claim 1, **characterised in that** the aqueous phase obtained in step **II**) is separated into water and alcohol, and the alcohol is recycled to step **I).**

3. The method according to one of claims 1 to 2, **characterised in that** the C₃- to C₅-hydrocarbon fraction is separated from part of the gas phase obtained in step **II)** and is combined with the liquid hydrocarbon phase obtained in step **II).**

4. The method according to one of claims 1 to 3, **characterised in that**, in order to produce an isoparaffin-rich gasoline, part of the heavy durene-containing aromatics fraction obtained in step **V)** is recycled to step **I),** or that the durene is crystallised out from the durene-containing heavy aromatics fraction obtained in step **V),** and part of the crystallised durene is dissolved in alcohol and/or gasoline hydrocarbons and recycled to step **I).**

5. The method according to one of claims 1 to 4, **characterised in that**, in order to produce an isoparaffin-rich gasoline, hydrogen is additionally fed into the tubular reactor in step **I).**

6. A device for the synthesis of synthetic gasoline with a high isoparaffin content in the range of 50-65 mass% and a low aromatics content in the range of 20-35 mass%, comprising the following components:
I. an isothermal tubular reactor **(R),** containing a catalyst, for the catalytic conversion of alcohols into a product mixture containing a hydrocarbon mixture and water,
wherein the catalyst is partially diluted in the inlet region of the alcohol with inert material with a mass fraction of 5 to 30 %, based on the total mass of catalyst and inert material,
II. a three-phase separator **(S)** for separating the product mixture obtained in the tubular reactor **(R)** into a liquid hydrocarbon phase, an aqueous phase containing unconverted alcohol, and a gas phase,
III. a first separation device **(K1)** which is suitable for separating a liquid hydrocarbon phase that contains hydrocarbons with 3 to 11 carbon atoms into a C₃-C₄ fraction, and a C₅₊ fraction,
IV. a second separation device **(K2),** which is suitable for separating a hydrocarbon phase that contains hydrocarbons with 5 to 11 carbon atoms into a durene-containing heavy aromatics fraction, and a stable gasoline fraction,
V. a connection line **(VL1)** from the tubular reactor **R** to the three-phase separator **S** for transporting the reaction product, which contains C1-C11 hydrocarbons, water, and alcohol,
VI. a connection line **(VL3)** from the three-phase separator **(S)** to the first separation device **(K1),** which is suitable for transporting a liquid hydrocarbon phase that contains hydrocarbons with 3 to 11 carbon atoms,
VII. a connection line **(VL5)** from the first separation device **(K1)** to the second separation device **(K2),** which is suitable for transporting a C₅₊ hydrocarbon fraction,
VIII. a return line **(RL1)** from the three-phase separator **(S)** to the tubular reactor **(R)** for recycling the gas phase separated off in the three-phase separator **(S)**
IX. a return line **(RL3)** from the first separation device **(K1)** to the tubular reactor **(R)** for recycling the C₃-C₄ hydrocarbon fraction,
wherein the components I. to IV. are connected in series, and
wherein the device contains a fourth separation device **(K4)** which is suitable for separating a gas phase containing C₁- to C₅- hydrocarbons into a C₁- to C₂-hydrocarbon fraction and a C₃- to C₅- hydrocarbon fraction, and
a connection line **(VL6)** from the three-phase separator **(S)** to the fourth separation device **(K4),** which is suitable for transporting a hydrocarbon gas that contains hydrocarbons with 1 to 5 carbon atoms, and
a connection line **(VL4)** from the fourth separation device **(K4)** to the first separation device **(K1),** which is suitable for transporting C₃- to C₅-hydrocarbons.

7. The device according to claim 6, **characterised in that** the device additionally contains a crystalliser **(KR1)** for crystallising out durene from the durene-containing heavy aromatics fraction, a connection line **(VL7)** from the separation device **(K2)** to the crystalliser **(KR1)** for transporting a heavy aromatics fraction, a dissolving device **(V1)** for dissolving part of the durene separated off in the crystalliser, and a return line **(RL4)** from the dissolving device **(V1)** to the tubular reactor **R** for recycling the dissolved durene.

8. The device according to one of claims 6 or 7, **characterised in that** the device additionally contains a return line **(RL5)** from the second separation device **(K2)** to the tubular reactor **(R)** for recycling part of the durene-containing aromatics fraction.

9. The device according to one of claims 6 to 8, **characterised in that** the device contains a third separation device **(K3)** which is suitable for separating an aqueous phase containing alcohol into water and alcohol, and that a connection line **(VL2)** from the three-phase separator **(S)** to the third separation device **(K3)** is arranged which is suitable for transporting an aqueous phase, which contains alcohol, and that a return line **(RL2)** from the third separation device **(K3)** to the tubular reactor **(R)** is arranged for recycling of the unconverted alcohol.

## Revendications

1. Procédé de production d'une essence de synthèse présentant une forte teneur en isoparaffines dans la plage comprise entre 50 et 65 % en masse et présentant une faible teneur en composés aromatiques dans la plage comprise entre 20 et 35 % en masse, comprenant les étapes suivantes :
I. la réaction catalytique d'alcool de départ dans un gaz de départ en un mélange de produits contenant de l'eau et un mélange d'hydrocarbures constitué d'oléfines, de n-paraffines, d'isoparaffines, de composés aromatiques et de naphtènes dans un réacteur tubulaire isotherme contenant un catalyseur,
où la température de réaction dans le réacteur tubulaire est comprise entre 300 et 370 °C,
où l'alcool de départ présente une teneur en eau inférieure à 20 % en masse,
II. la séparation du mélange de produits obtenu à l'étape **I**) en
- une phase d'hydrocarbures liquide,
- une phase aqueuse contenant l'alcool n'ayant pas réagi,
et
- une phase gazeuse contenant des hydrocarbures en C₁- à Cs-,
III. le recyclage de la phase gazeuse obtenue à l'étape **II**) à l'étape **I**),
IV. la séparation de la phase d'hydrocarbures liquide obtenue à l'étape **II**) en
- une fraction d'hydrocarbures en C₃- et C₄- contenant des oléfines et
- une fraction d'hydrocarbures d'essence (= fraction C₅₊-) contenant une fraction lourde de composés aromatiques contenant du durène,
V. la séparation de la fraction d'hydrocarbures d'essence obtenue à l'étape **IV)** en une fraction lourde de composés aromatiques contenant du durène et une fraction d'essence stabilisée,
où à l'étape **I**) le chargement d'alcool du catalyseur est de 2 à 5 m³ _{alcool}/hm³_{cat},
où, produire une essence riche en isoparaffines, le catalyseur, dans la zone d'entrée de l'alcool, est partiellement dilué avec 5 à 30 % en masse de matériau inerte, rapporté à la masse totale de catalyseur et de matériau inerte afin de , et
où la fraction d'hydrocarbures en C₃- à C₄- contenant des oléfines séparée à l'étape **IV)** est réintroduite comme matière de départ à l'étape **I**),
où à l'étape **I**) la fraction molaire de l'alcool à faire réagir à l'entrée du réacteur tubulaire comprend 25 à 50 % en moles, mesurée sur la quantité molaire totale du gaz d'alimentation, comprenant l'alcool d'alimentation et les composants réintroduits.

2. Procédé selon la revendication 1, **caractérisé en ce que** la phase aqueuse obtenue à l'étape **II)** est séparée en eau et alcool et l'alcool est réintroduit à l'étape **I**).

3. Procédé selon l'une des revendications 1 à 2, **caractérisé en ce que** la fraction d'hydrocarbures en C₃- à C₅- est séparée d'une partie de la phase gazeuse obtenue à l'étape **II)** et celle-ci est combinée avec la phase d'hydrocarbures liquide obtenue à l'étape II).

4. Procédé selon l'une quelconque des revendications 1 à 3, **caractérisé en ce que**, pour produire une essence riche en isoparaffines, une partie de la fraction lourde de composés aromatiques contenant du durène obtenu à l'étape **V)** est réintroduite à l'étape **I**) ou que le durène est cristallisé à partir de la fraction lourde de composés aromatiques contenant du durène obtenue à l'étape **V)** et une partie du durène cristallisé est dissoute dans de l'alcool et/ou des hydrocarbures d'essence et réintroduite à l'étape **I**).

5. Procédé selon l'une quelconque des revendications 1 à 4, **caractérisé en ce que**, pour produire une essence riche en isoparaffines, de l'hydrogène est en outre introduit dans le réacteur tubulaire à l'étape **I**).

6. Installation de synthèse d'essence de synthèse présentant une forte teneur en isoparaffines dans la plage comprise entre 50 et 65 % en masse et présentant une faible teneur en composés aromatiques dans la plage comprise entre 20 et 35 % en masse, comprenant les composants suivants :
I. un réacteur tubulaire isotherme **(R)** contenant un catalyseur de réaction catalytique d'alcools en un mélange de produits contenant un mélange d'hydrocarbures et d'eau,
où le catalyseur dans la zone d'entrée de l'alcool est partiellement dilué avec 5 à 30 % en masse de matériau inerte, rapporté à la masse totale de catalyseur et de matériau inerte,
II. un séparateur triphasique **(S)** pour séparer le mélange de produits obtenu dans le réacteur tubulaire **(R)** en une phase d'hydrocarbures liquide, une phase aqueuse contenant de l'alcool n'ayant pas réagi et une phase gazeuse,
III. un premier dispositif de séparation **(K1),** lequel est conçu pour séparer une phase d'hydrocarbures liquide contenant des hydrocarbures de 3 à 11 atomes de carbone en une fraction C₃-C₄- et une fraction C₅₊-,
IV. un deuxième dispositif de séparation **(K2),** lequel est conçu pour séparer une phase d'hydrocarbures contenant des hydrocarbures de 5 à 11 atomes de carbone en une fraction lourde de composés aromatiques contenant du durène et une fraction d'essence stabilisée,
V. une conduite de liaison **(VL1)** du réacteur tubulaire **R** au séparateur triphasique **S** pour transporter le produit de réaction contenant des hydrocarbures en C₁-C₁₁-, de l'eau et de l'alcool,
VI. une conduite de liaison **(VL3)** du séparateur triphasique **(S)** au premier dispositif de séparation **(K1),** laquelle est conçue pour transporter une phase d'hydrocarbures liquide contenant des hydrocarbures de 3 à 11 atomes de carbone,
VII. une conduite de liaison **(VL5)** du premier dispositif de séparation **(K1)** au deuxième dispositif de séparation **(K2),** laquelle est conçue pour transporter une fraction d'hydrocarbures en C₅₊-,
VIII. une conduite de recyclage **(RL1)** du séparateur triphasique **(S)** au réacteur tubulaire **(R)** pour réintroduire la phase gazeuse séparée dans le séparateur triphasique **(S),**
IX. ainsi qu'une conduite de recyclage **(RL3)** du premier dispositif de séparation **(K1)** au réacteur tubulaire **(R)** pour réintroduire la fraction d'hydrocarbures C₃-C4-,
où les composants I. à IV. sont connectés en série, et
où l'installation contient un quatrième dispositif de séparation **(K4),** lequel est conçu pour séparer une phase gazeuse contenant des hydrocarbures en C₁- à C₅-en une fraction d'hydrocarbures en C₁- à C₂- et en C₃- à C₅-, et
une conduite de liaison **(VL6)** du séparateur triphasique **(S)** au quatrième dispositif de séparation **(K4),** laquelle est conçue pour transporter un gaz d'hydrocarbures contenant des hydrocarbures de 1 à 5 atomes de carbone et
une conduite de liaison **(VL4)** du quatrième dispositif de séparation **(K4)** au premier dispositif de séparation **(K1),** laquelle est conçue pour transporter les hydrocarbures en C₃- à C₅-.

7. Installation selon la revendication 6, **caractérisée en ce que** l'installation comporte également un cristallisoir **(KR1)** pour cristalliser le durène à partir de la fraction lourde de composés aromatiques contenant du durène, une conduite de liaison **(VL7)** du dispositif de séparation **(K2)** au cristallisoir **(KR1)** pour transporter une fraction lourde de composés aromatiques, un dispositif **(V1)** de dissolution d'une partie du durène séparé dans le cristallisoir et une conduite de réintroduction **(RL4)** du dispositif **(V1)** au réacteur tubulaire **(R)** pour réintroduire du durène dissous.

8. Installation selon la revendication 6 ou 7, **caractérisée en ce que** l'installation comporte en outre une conduite de recyclage **(RL5)** du deuxième dispositif de séparation **(K2)** au réacteur tubulaire **(R)** pour réintroduire une partie de la fraction de composés aromatiques contenant du durène.

9. Installation selon l'une quelconque des revendications 6 à 8, **caractérisée en ce que** l'installation comporte un troisième dispositif de séparation **(K3),** lequel est conçu pour séparer une phase aqueuse contenant de l'alcool en eau et alcool et qu'une conduite de liaison **(VL2)** du réseau triphasique séparateur **(S)** au troisième dispositif de séparation **(K3)** est conçue pour transporter une phase aqueuse contenant de l'alcool, et qu'une conduite de recyclage **(RL2)** du troisième dispositif de séparation **(K3)** au réacteur tubulaire **(R)** est conçue pour recycler l'alcool n'ayant pas réagi.
